(19) Europäisches Patentamt
European Patent Office
Office européen des brevets

(11) Veröffentlichungsnummer: **0 256 326 A1**

(12) # EUROPÄISCHE PATENTANMELDUNG

(21) Anmeldenummer: **87110468.3**

(51) Int. Cl.⁴: **C07C 131/00 , A01N 37/50**

(22) Anmeldetag: **20.07.87**

(30) Priorität: **28.07.86 DE 3625460**

(43) Veröffentlichungstag der Anmeldung:
**24.02.88 Patentblatt 88/08**

(84) Benannte Vertragsstaaten:
**AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Lunkenheimer, Winfried, Dr.**
**Bismarckstrasse 29**
**D-5600 Wuppertal 1(DE)**
Erfinder: **Berg, Dieter, Dr.**
**Gellertweg 27**
**D-5600 Wuppertal 1(DE)**
Erfinder: **Brandes, Wilhelm, Dr.**
**Eichendorffstrasse 3**
**D-5653 Leichlingen(DE)**

(54) **N-(2-Cyan-2-oximinoacetyl)-aminonitrile.**

(57) N-(2-Cyan-2-oximinoacetyl)-aminonitrile der allgemeinen Formel

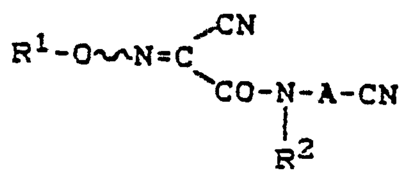

in welcher
$R^1$ für gegebenenfalls substituiertes Cycloalkyl oder die Gruppierung

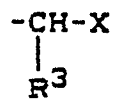 steht,

wobei
$R^3$ für Wasserstoff oder Alkyl steht und
X für Wasserstoff, Alkyl, Alkenyl, Alkinyl, Cyano, Hydroxycarbonyl, Alkoxcarbonyl, Aminocarbonyl, Alkylaminocarbonyl, Dialkylaminocarbonyl, für gegebenenfalls substituiertes Cycloalkyl, für gegebenenfalls substituiertes Aryl, für einen gegebenenfalls mit einem Benzolring annellierten Heterocyclus sowie für die Gruppierung

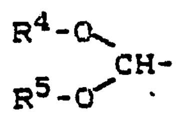

EP 0 256 326 A1

steht, wobei
$R^4$ für Alkyl steht und
$R^5$ für Alkyl steht oder
$R^4$ und $R^5$ gemeinsam mit dem Methylendioxy-Rest, an den sie gebunden sind, für einen 5-oder 6-gliedrigen Heterocyclus stehen,
$R^2$ für Wasserstoff oder Alkyl steht oder mit einem C-Atom aus der gegebenenfalls substituierten Alkylenkette A gemeinsam mit dem N-Atom, an das sie gebunden sind, für einen Heterocyclus stehen, und
A für eine gegebenenfalls substituierte Alkylenkette steht,
sowie deren physiologisch verträgliche Metallsalz-Komplexe, sowie Verfahren zer Herstellung dieser Verbindungen und deren Verwendung als Fungizide.

## N-(2-Cyan-2-oximinoacetyl)-aminonitrile

Die vorliegende Erfindung betrifft neue N-(2-Cyan-2-oximinoacetyl)-aminonitrile, mehrere Verfahren zu ihrer Herstellung und ihre Verwendung als Schädlingsbekämpfungsmittel, insbesondere als Fungizide.

Es ist bereits bekannt, daß bestimmte Verbindungen wie beispielsweise Zink-ethylen-1,2-bis-dithiocarbamat gute fungizide Wirksamkeit besitzen (vgl. US-PS 2 457 674). Die Wirkung dieser Verbindung ist jedoch, insbesondere bei niedrigen Aufwandmengen und -konzentrationen, nicht immer voll befriedigend.

Es wurden neue N-(2-Cyan-2-oximinoacetyl)-aminonitrile der allgemeinen Formel (I)

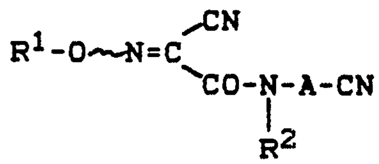

(I)

in welcher

$R^1$ für gegebenenfalls substituiertes Cycloalkyl oder die Gruppierung

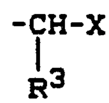

steht, wobei

$R^3$ für Wasserstoff oder Alkyl steht und

X für Wasserstoff, Alkyl, Alkenyl, Alkinyl, Cyano, Hydroxycarbonyl, Alkoxycarbonyl, Aminocarbonyl, Alkylaminocarbonyl, Dialkylaminocarbonyl, für gegebenenfalls substituiertes Cycloalkyl, für gegebenenfalls substituiertes Aryl, für einen gegebenenfalls mit einem Benzolring annellierten Heterocyclus sowie für die Gruppierung

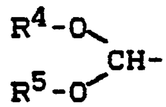

steht, wobei

$R^4$ für Alkyl steht und

$R^5$ für Alkyl steht oder

$R^4$ und $R^5$ gemeinsam mit dem Methylendioxy-Rest, an den sie gebunden sind, für einen 5-oder 6-gliedrigen Heterocyclus stehen,

$R^2$ für Wasserstoff oder Alkyl steht oder mit einem C-Atom aus der gegebenenfalls substituierten Alkylenkette A gemeinsam mit dem N-Atom, an das sie gebunden sind, für einen Heterocyclus steht, und

A für eine gegebenenfalls substituierte Alkylenkette steht,

sowie deren physiologisch verträgliche Metallsalz-Komplexe gefunden.

Die Verbindungen der Formel (I) besitzen für den Fall, daß A für eine substituierte Alkylenkette steht, mindestens ein asymmetrisches Kohlenstoffatom, sie können somit als optische Isomere vorliegen, die in unterschiedlichen Mengenverhältnissen anfallen können. Vorwiegend fallen sie als Racemate an. Ferner können die Verbindungen der Formel (I), bedingt durch die Oximinogruppe als E-und Z-Isomere sowie als E/Z-Isomerengemische auftreten.

Weiterhin wurde gefunden, daß sich die neuen N-(2-Cyan-2-oximinoacetyl)-aminonitrile der allgemeinen Formel (I)

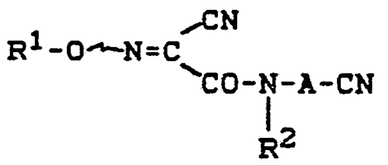 (I)

in welcher
$R^1$ für Cycloalkyl oder die Gruppierung

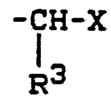

steht, wobei
$R^3$ für Wasserstoff oder Alkyl steht und
X für Wasserstoff, Alkyl, Alkenyl, Alkinyl, Cyano, Hydroxycarbonyl, Alkoxycarbonyl, Aminocarbonyl, Alkylaminocarbonyl, Dialkylaminocarbonyl, für gegebenenfalls substituiertes Cycloalkyl, für gegebenenfalls substituiertes Aryl, für einen gegebenenfalls mit einem Benzolring annellierten Heterocyclus sowie für die Gruppierung

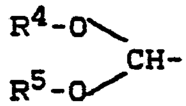 steht, wobei

$R^4$ für Alkyl steht und
$R^5$ für Alkyl steht oder
$R^4$ und $R^5$ gemeinsam mit dem Methylendioxy-Rest, an den sie gebunden sind, für einen 5-oder 6-gliedrigen Heterocyclus stehen,
$R^2$ für Wasserstoff oder Alkyl steht oder
$R^2$ mit einem C-Atom aus der gegebenenfalls substituierten Alkylenkette A gemeinsam mit dem N-Atom, an das sie gebunden sind, für einen Heterocyclus steht, und
A für eine gegebenenfalls substituierte Alkylenkette steht,
sowie deren physiologisch verträgliche Metallsalz-Komplexe nach folgenden Verfahren herstellen lassen:

Man erhält,

a) die erfindungsgemäßen N-[(E)-2-Cyan-2-oximinoacetyl]-aminonitrile der allgemeinen Formel (I)

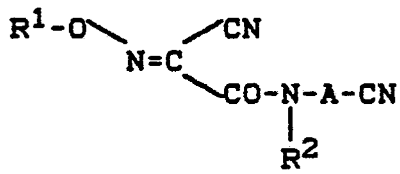 (I)

in welcher
$R^1$, $R^2$ und A die oben angegebenen Bedeutungen haben,
wenn man die carboxy-aktivierten Derivate der (E)-2-Cyan-2-oximino-essigsäuren der allgemeinen Formel (II)

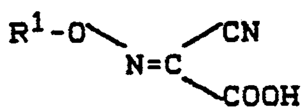 (II)

in welcher
$R^1$ die oben angegebene Bedeutung hat,
mit Aminonitrilen der allgemeinen Formel (III)

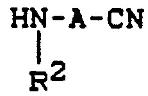 (III)

in welcher
$R^2$ und A die oben angegebenen Bedeutungen haben,
gegebenenfalls in Gegenwart eines Katalysators, gegebenenfalls in Gegenwart eines Säurebindemittels und gegebenenfalsl in Gegenwart eines Verdünnungsmittels umsetzt,
oder man erhält,
b) die erfindungsgemäßen N-[(E)-2-Cyan-2-oximinoacetyl]-aminonitrile der allgemeinen Formel (I)

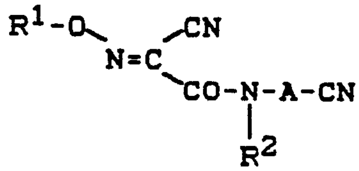 (I)

in welcher
$R^1$, $R^2$ und A die oben angegebenen Bedeutungen haben,
wenn man die E-Oxime der allgemeinen Formel (IV)

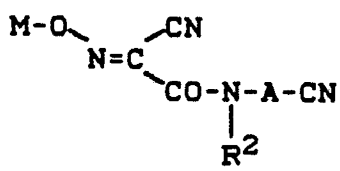 (IV)

in welcher
$R^2$ und A die oben angegebenen Bedeutungen haben und
M für Wasserstoff oder ein Alkalimetall, wie Natrium oder Kalium steht,
mit Verbindungen der allgemeinen Formel (V)
$R^1$-W (V)
in welcher
$R^1$ die oben angegebene Bedeutungen hat und
W für Halogen oder einen Sulfonyl(di)oxy-Rest steht,
gegebenenfalls in Gegenwart einer Base und in Gegenwart eines Lösungsmittels umsetzt,
oder man erhält,
c) die erfindungsgemäßen N-[(Z)-2-Cyano-2-oximinoacetyl]-aminonitrile der allgemeinen Formel (I-1)

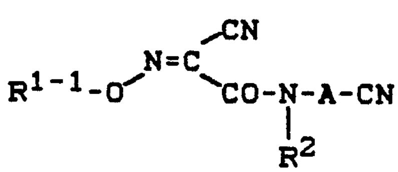 (I-1)

in welcher
$R^2$ und A die oben angegebenen Bedeutungen haben und
$R^{1-1}$ für gegebenenfalls substituiertes Cycloalkyl oder die Gruppierung

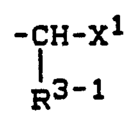

steht, wobei

$R^{3-1}$ für Wasserstoff oder Alkyl steht und

$X^1$ für Wasserstoff, Alkyl, Alkenyl, Alkinyl, Cyano, für gegebenenfalls substituiertes Cycloalkyl, für gegebenenfalls substituiertes Aryl oder für einen gegebenenfalls mit einem Benzolring annellierten Heterocyclus steht,

wenn man (Z)-2-Cyan-2-oximino-essigsäurederivate der allgemeinen Formel (VI)

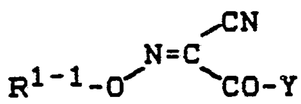

(VI)

in welcher

$R^{1-1}$ die oben angegebene Bedeutung hat, und

Y für Cl oder Alkoxy (vorzugsweise mit 1-4 C-Atomen) steht,

mit Aminonitrilen der allgemeinen Formel (III)

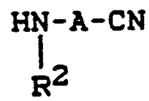

(III)

in welcher

$R^2$ und A die oben angegebenen Bedeutungen haben,

gegebenenfalls in Gegenwart eines Katalysators, gegebenenfalls in Gegenwart eines Säurebindemittels und gegebenenfalls in Gegenwart eines Verdünnungsmittels umsetzt,

oder man erhält

d) die erfindungsgemäßen N-[(E)-2-Cyan-2-oximinoacetyl]-aminonitrile der allgemeinen Formel (I-2)

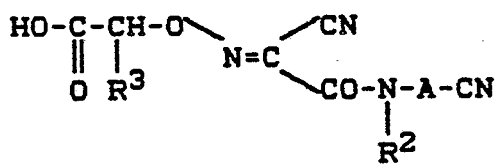

(I-2)

in welcher

$R^2$, $R^3$ und A die oben angegebenen Bedeutungen haben,

wenn man die erfindungsgemäßen N-[(E)-2-Cyan-2-oximinoacetyl]-aminonitrile der allgemeinen Formel (I-3)

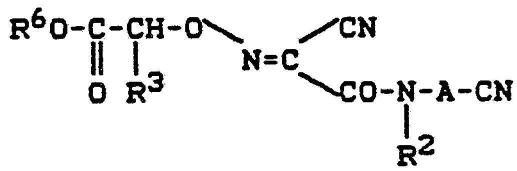

(I-3)

in welcher

$R^2$, $R^3$ und A die oben angegebenen Bedeutungen haben und

$R^6$ für Alkyl steht,

mit Alkali-oder Erdalkalihydroxiden in einem Verdünnungsmittel hydrolisiert,

oder man erhält

e) die erfindungsgemäßen N-[(E)-2-Cyan-2-oximinoacetyl]-aminonitrile der allgemeinen Formel (I-4)

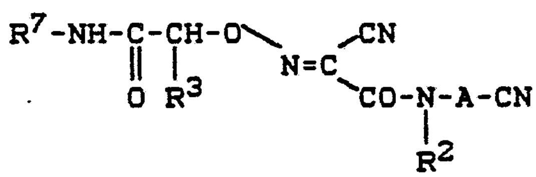

(I-4)

in welcher
$R^2$, $R^3$ und A die oben angegebenen Bedeutungen haben und
$R^7$ für Wasserstoff oder Alkyl steht,
wenn man die erfindungsgemäßen N-[(E)-2-Cyan-2-oximinoacetyl]-aminonitrile der allgemeinen Formel (I-3)

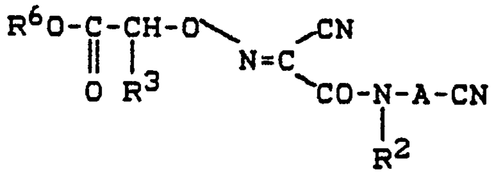
(I-3)

in welcher
$R^2$, $R^3$, $R^6$ und A die oben angegebenen Bedeutungen haben,
mit Aminen der allgemeinen Formel (VII)
$H_2N\text{-}R^7$ (VII)
in welcher
$R^7$ die oben angegebene Bedeutung hat,
in Gegenwart eines Lösungsmittels umsetzt,
oder man erhält
f) die erfindungsgemäßen N-[(E)-2-Cyan-2-oximinoacetyl]-aminonitrile der allgemeinen Formel (I-2)

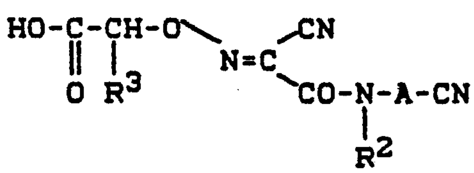
(I-2)

in welcher
$R^2$, $R^3$ und A die oben angegebenen Bedeutungen haben,
wenn man die erfindungsgemäßen N-[(E)-2-Cyan-2-oximinoacetyl]-aminonitrile der allgemeinen Formel (I-5)

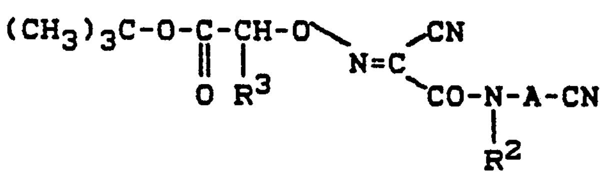
(I-5)

in welcher
$R^2$, $R^3$ und A die oben angegebenen Bedeutungen haben,
mit einer Säure, gegebenenfalls in Gegenwart eines Lösungsmittels behandelt,
oder man erhält
g) die erfindungsgemäßen N-[(E)-2-Cyan-2-oximinoacetyl]-aminonotrile der allgemeinen Formel (I-6)

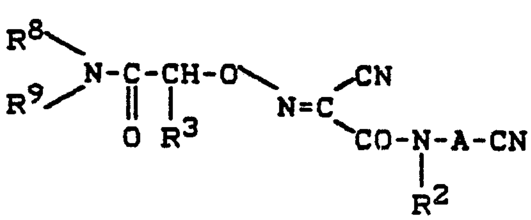
(I-6)

in welcher
$R^2$, $R^3$ und A die oben angegebenen Bedeutungen haben,
$R^8$ für Wasserstoff oder Alkyl steht und
$R^9$ für Wasserstoff oder Alkyl steht,

wenn man carboxy-aktivierte Derivate der erfindungsgemäßen N-[(E)-2-Cyan-2-oximinoactyl]-aminonitrile der allgemeinen Formel (I-2)

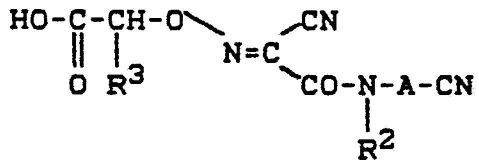

(I-2)

in welcher
$R^2$, $R^3$ und A die oben angegebenen Bedeutungen haben,
mit Aminen der allgemeinen Formel (VIII)
$HNR^8R^9$ (VIII)
in welcher
$R^8$ und $R^9$ die oben angegebenen Bedeutungen haben,
gegebenenfalls in Gegenwart einer Base, in Gegenwart eines Verdünnungsmittels, und gegebenenfalls in Gegenwart eines Katalysators umsetzt.

An die so erhaltenen Verbindungen der Formel (I) kann gegebenenfalls anschließend ein Metallsalz addiert werden.

Schließlich wurde gefunden, daß die neuen N-(2-Cyan-2-oximinoactyl)-aminonitrile der allgemeinen Formel (I) sowie deren Metallsalz-Komplexe insbesondere starke fungizide Eigenschaften aufweisen. Dabei zeigen überraschenderweise die erfindungsgemäßen Verbindungen eine höhere Wirkung als die aus dem Stand der Technik bekannten Verbindungen, wie bei spielsweise das Zink-ethylen-1,2-bis-dithiocarbamat, welches eine wirkungsmäßig naheliegende Verbindung ist. Die erfindungsgemäßen Stoffe stellen somit eine Bereicherung der Technik dar.

Die erfindungsgemäßen N-(2-Cyan-2-oximinoactyl)-aminonitrile sind durch die allgemeinen Formel (I) allgemein definiert. Bevorzugt sind Verbindungen der allgemeinen Formel (I), in denen
$R^1$ für gegebenenfalls ein-bis dreifach, gleich oder verschieden durch Halogen oder Methyl substituiertes Cycloalkyl mit 3 bis 6 Kohlenstoffatomen oder für die Gruppierung

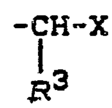

steht, wobei
$R^3$ für Wasserstoff oder Methyl steht und
X für Wasserstoff, für geradkettiges oder verzweigtes Alkyl mit 1 bis 8 Kohlenstoffatomen, für Vinyl, Ethinyl, Cyano, Hydroxycarbonyl, für Alkoxycarbonyl mit 1 bis 4 Kohlenstoffatomen im Alkoxyteil, für Aminocarbonyl, Alkylaminocarbonyl und Dialkylaminocarbonyl mit jeweils 1 bis 4 Kohlenstoffatomen in jedem Alkylteil, für gegebenenfalls ein-oder zweifach, gleich oder verschieden durch Halogen substituiertes Cyclopropyl, für gegebenenfalls durch Halogen oder Alkyl mit 1 bis 4 Kohlenstoffatomen substituiertes Phenyl, für einen gegebenenfalls mit einem Benzolring annellierten 5-oder 6-gliedrigen Heterocyclus, welcher 1 bis 3 gleiche oder verschiedene Heteroatome aus der Gruppe Stickstoff, Sauerstoff und Schwefel enthält oder für den Rest

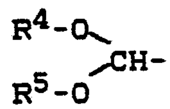

steht, wobei
$R^4$ für Methyl oder Ethyl steht und
$R^5$ für Methyl oder Ethyl steht oder
$R^4$ und $R^5$ gemeinsam mit dem Methylendioxy-Rest, an den sie gebunden sind, für einen 5-oder 6-gliedrigen Heterocyclus stehen,

$R^2$ für Wasserstoff oder für geradkettiges oder verzweigtes Alkyl mit 1 bis 4 Kohlenstoffatomen steht oder
$R^2$ mit einem C-Atom aus der gegebenenfalls substituierten Alkylenkette A gemeinsam mit dem N-Atom, an das sie gebunden sind, für einen 5-oder 6-gliedrigen Heterocyclus steht und
A für eine gegebenenfalls bis zu dreifach, gleich oder verschieden substituierte Alkylenkette mit 1 bis 5 Kohlenstoffatomen steht, wobei als Substituenten genannt seien: gegebenenfalls bis zu vierfach, gleich oder verschieden substituiertes geradkettiges oder verzweigtes Alkyl mit 1 bis 8 Kohlenstoffatomen, wo bei als Substituenten Halogen, Hydroxy, sowie Alkoxy mit 1 bis 4 Kohlenstoffatomen in Frage kommen, ferner gegebenenfalls im Arylteil einfach bis vierfach, gleich oder verschieden substituiertes Aryl oder Aralkyl mit 1 bis 4 Kohlenstoffatomen im Alkylteil und 6 bis 10 Kohlenstoffatomen im Arylteil, wobei als Substituenten Halogen, Hydroxy, Nitro sowie Alkyl und Alkoxy mit jeweils 1 bis 4 Kohlenstoffatomen genannt seien, ferner jeweils gegebenenfalls durch Alkyl mit 1 bis 4 Kohlenstoffatomen substituiertes Cycloalkyl mit 3 bis 7 Kohlenstoffatomen oder Cycloalkyl mit 4 bis 7 Kohlenstoffatomen, sowie ein 5-oder 6-gliedriger Heterocyclus, welcher 1 bis 3 gleiche oder verschiedene Heteroatome aus der Gruppe Stickstoff, Sauerstoff und Schwefel enthält.

Besonders bevorzugt sind diejenigen Verbindungen der allgemeinen Formel (I), in denen
$R^1$ für gegebenenfalls ein-oder zweifach, gleich oder verschieden durch Chlor oder Methyl substituiertes Cyclopentyl oder Cyclohexyl oder für die Gruppierung

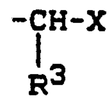

steht, wobei
$R^3$ für Wasserstoff oder Methyl steht und
X für Wasserstoff, Methyl, Ethyl, Vinyl, Ethinyl, Cyano, Hydroxycarbonyl, Metoxycarbonyl, Ethoxycarbonyl, tert.-Butoxycarbonyl, Aminocarbonyl, Methylaminocarbonyl, Dimethylaminocarbonyl, für gegebenenfalls ein -oder zweifach durch Chlor substituiertes Cyclopropyl, für gegebenenfalls durch Chlor, Methyl oder Ethyl substituiertes Phenyl, für Pyrazol-1-yl, 1,2,4-Triazol-1-yl, für die Reste

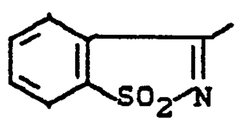 und 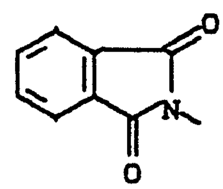 oder

für den Rest

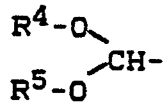

steht, wobei
$R^4$ für Methyl oder Ethyl steht und
$R^5$ für Methyl oder Ethyl steht oder
$R^4$ und $R^5$ gemeinsam mit dem Methylendioxy-Rest, an den sie gebunden sind, für einen 5-oder 6-gliedrigen Heterocyclus stehen,
$R^2$ für Wasserstoff oder Methyl steht oder
$R^2$ mit einem C-Atom der gegebenenfalls substituierten Alkylenkette A gemeinsam mit dem N-Atom, an das sie gebunden sind, für einen 5-oder 6-gliedrigen Heterocyclus steht und
A für gegebenenfalls einfach substituiertes Methylen oder Ethylen steht, wobei als Substituenten insbesondere genannt seien: gegebenenfalls einfach substituiertes geradkettiges oder verzweigtes Alkyl mit 1 bis 4 Kohlenstoffatomen, wobei als Substituenten Chlor, Brom, Hydroxy sowie Methoxy in Frage kommen, ferner gegebenenfalls ein im Phenylteil ein-bis dreifach, gleich oder verschieden substituiertes Phenyl oder Phenylalkyl mit 1 oder 2 Kohlenstoffatomen im Alkylteil, wobei als Substituenten Chlor, Hydroxy, Nitro, Methyl sowie Methoxy genannt seien, ferner jeweils gegebenenfalls durch Methyl oder Ethyl substituiertes

Cyclopropyl, Cylopentyl, Cyclohexyl, Cyclopentenyl und Cyclohexenyl, ferner 2-, 3-oder 4-Pyridyl, 2-oder 3-Thienyl sowie 2-oder 3-Furyl.

Die Ausgangs-bzw. Endprodukte in den Verfahren a) bis g) sind durch die allgemeinen Formeln (I) bis (I-6) und (II) bis (VIII) allgemein definiert.

Bevorzugt sind Verbindungen der allgemeinen Formeln (I) bis (I-6) und (II) bis (VIII), in denen
$R^1$ für gegebenenfalls ein-bis dreifach, gleich oder verschieden durch Halogen oder Methyl substituierten Cycloalkyl mit 3 bis 6 Kohlenstoffatomen oder für die Gruppierung

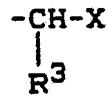

steht, wobei
$R^3$ für Wasserstoff oder Methyl steht und
X für Wasserstoff, für geradkettiges oder verzweigtes Alkyl mit 1 bis 8 Kohlenstoffatomen, für Vinyl, Ethinyl, Cyano, Hydroxycarboxyl, für Alkoxycarbonyl mit 1 bis 4 Kohlenstoffatomen im Alkoxyteil, für Aminocarbonyl, Alkylaminocarbonyl und Dialkylaminocarbonyl mit jeweils 1 bis 4 Kohlenstoffatomen in jedem Alkylteil, für ge gebenenfalls ein-oder zweifach durch Halogen substituiertes Cyclopropyl, für einen gegebenenfalls mit einem Benzolring annellierten 5-oder 6-gliedrigen Heterocyclus, welcher 1 bis 3 gleiche oder verschiedene Heteroatome aus der Gruppe Stickstoff, Sauerstoff und Schwefel enthält oder für den Rest

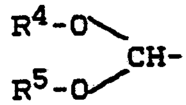

steht, wobei
$R^4$ für Methyl oder Ethyl steht, und
$R^5$ für Methyl oder Ethyl steht oder
$R^4$ und $R^5$ gemeinsam mit dem Methylendioxy-Rest, an den sie gebunden sind, für einen 5-oder 6-gliedrigen Heterocyclus stehen,
$R^{1-1}$ für gegebenenfalls ein-bis dreifach, gleich oder verschieden durch Halogen oder Methyl substituiertes Cycloalkyl mit 3 bis 6 Kohlenstoffatomen oder für die Gruppierung

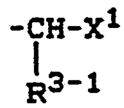

steht, wobei
$R^{3-1}$ für Wasserstoff oder Methyl steht und
$X^1$ für Wasserstoff, für geradkettiges oder verzweigtes Alkyl mit 1 bis 8 Kohlenstoffatomen, für Vinyl, Ethinyl, Cyano, für gegebenenfalls ein-oder zweifach durch Halogen substituiertes Cyclopropyl, für gegebenenfalls durch Halogen oder Alkyl mit 1 bis 4 Kohlenstoffatomen substituiertes Phenyl, für einen gegebenenfalls mit einem Benzolring annellierten 5-oder 6-gliedrigen Heterocyclus, welcher 1 bis 3 gleiche oder verschiedene Heteroatome aus der Gruppe Stickstoff, Sauerstoff und Schwefel enthält, steht,
$R^2$ für Wasserstoff sowie für geradkettiges oder verzweigtes Alkyl mit 1 bis 4 Kohlenstoffatomen steht oder
$R^2$ mit einem C-Atom aus der gegebenenfalls substituierten Alkylenkette A gemeinsam mit dem N-Atom, an das sie gebunden sind, für einen 5-oder 6-gliedrigen Heterocyclus steht,
A für eine gegebenenfalls bis zu dreifach substituierte Alkylenkette mit 1 bis 5 Kohlenstoffatomen steht wobei als Substituenten genannt seien: gegebenenfalls bis zu vierfach, gleich oder verschieden, substituiertes geradkettiges oder verzweigtes Alkyl mit 1 bis 8 Kohlenstoffatomen, wobei als Substituenten Halogen, Hydroxy, sowie Alkoxy mit 1 bis 4 Kohlenstoffatomen in Frage kommen, ferner gegebenenfalls im Arylteil einfach bis vierfach, gleich oder verschieden substituiertes Aryl oder Aralkyl mit 1 bis 4 Kohlenstoffatomen im Alkylteil, wobei als Substituenten Halogen, Hydroxy, Nitro sowie Alkyl und Alkoxy mit jeweils mit 1 bis 4 Kohlenstoffatomen genannt seien, ferner jeweils gegebenenfalls durch Alkyl mit 1 bis 4 Kohlenstoffatomen substituiertes Cycloalkyl mit 3 bis 7 Kohlenstoffatomen oder Cycloalkenyl mit 4 bis 7 Kohlenstoffatomen oder ein 5-oder 6-gliedriger Heterocyclus, welcher1 bis 3 gleiche oder verschiedene Heteroatome aus der

Gruppe Stickstoff, Sauerstoff und Schwefel enthält,
$R^6$ für Methyl oder Ethyl steht,
$R^7$ für Wasserstoff oder für geradkettiges oder verzweigtes Alkyl mit 1 bis 4 Kohlenstoffatomen steht,
$R^8$ für Wasserstoff oder für geradkettiges oder verzweigtes Alkyl mit 1 bis 4 Kohlenstoffatomen steht,
$R^9$ für Wasserstoff oder für geradkettiges oder verzweigtes Alkyl mit 1 bis 4 Kohlenstoffatomen steht,
M für Wasserstoff, Natrium oder Kalium steht und
W für Halogen oder einen Sulfonyl(di)oxy-Rest, wie vorzugsweise Chlor, Brom, Iod, $-OSO_2OCH_3$, $-OSO_2OC_2H_5$, $-OSO_2CH_3$ oder

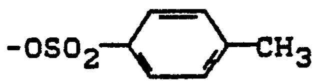

steht.

Besonders bevorzugt sind diejenigen Verbindungen der allgemeinen Formel (I) bis (I-6) und (II) bis (VIII), in denen
$R^1$ für gegebenenfalls ein-oder zweifach, gleich oder verschieden durch Chlor oder Methyl substituiertes Cyclopentyl oder Cyclohexyl oder für die Gruppierung

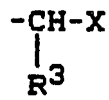

steht, wobei
$R^3$ für Wasserstoff oder Methyl steht und
X für Wasserstoff, Methyl, Ethyl, Vinyl, Ethinyl, Cyano, Hydroxycarbonyl, Methoxycarbonyl, Ethoxycarbonyl, tert.-Butoxycarbonyl, Aminocarbonyl, Methylaminocarbonyl, Dimethylaminocarbonyl, für gegebenenfalls ein- oder zweifach durch Chlor substituiertes Cyclopropyl für Pyrazol-1-yl, 1,2,4-Triazol-1-yl, für die Reste

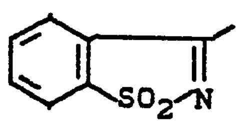 und 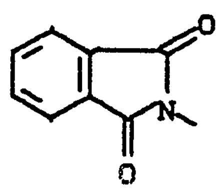 sowie

für den Rest

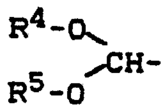

steht, wobei
$R^4$ für Methyl oder Ethyl steht und
$R^5$ für Methyl oder Ethyl steht oder
$R^4$ und $R^5$ gemeinsam mit dem Methylendioxy-Rest, an den sie gebunden sind, für einen 5-oder 6-gliedrigen Heterocyclus stehen,
$R^{1-1}$ für gegebenenfalls ein-oder zweifach, gleich oder verschieden durch Chlor oder Methyl substituiertes Cyclopentyl oder Cyclohexyl oder für die Gruppierung

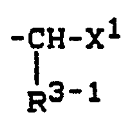

steht, wobei
$R^{3-1}$ für Wasserstoff oder Methyl steht und

$X^1$ für Wasserstoff, Methyl, Ethyl, Vinyl, Ethinyl, Cyano, für gegebenenfalls ein-oder zweifach durch Chlor substituiertes Cyclopropyl, für gegebenenfalls durch Chlor, Methyl oder Ethyl substituiertes Phenyl, für Pyrazol-1-yl, 1,2,4-Triazol-1-yl sowie für die Reste

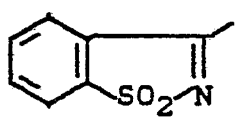 und 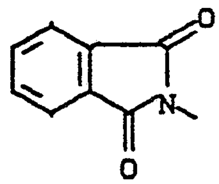 steht,

$R^2$ für Wasserstoff oder Methyl steht oder
$R^2$ mit einem C-Atomen der gegebenenfalls substituierten Alkylenkette A gemeinsam mit dem N-Atom, an das sie gebunden sind, für einen 5-oder 6-gliedrigen Heterocyclus steht,
A für gegebenenfalls einfach substituiertes Methylen oder Ethylen steht, wobei als Substituenten insbesondere genannt seien: gegebenenfalls einfach substituiertes geradkettiges oder verzweigtes Alkyl mit 1 bis 4 Kohlenstoffatomen, wobei als Substituenten Chlor, Brom, Hydroxy sowie Methoxy in Frage kommen, ferner gegebenenfalls im Phenylteil ein-bis dreifach, gleich oder verschieden substituiertes Phenyl oder Phenylalkyl mit 1 oder 2 C-Atomen im Alkylteil, wobei als Substituenten Chlor, Hydroxy, Nitro, Methyl sowie Methoxy genannt seien, ferner jeweils gegebenenfalls durch Methyl oder Ethyl substituiertes Cyclopropyl, Cyclopentyl, Cyclohexyl, Cyclopentenyl und Cyclohexenyl, ferner 2-, 3-oder 4-Pyridyl, 2-oder 3-Thienyl sowie 2-oder 3-Furyl steht,
$R^6$ für Methyl oder Ethyl steht,
$R^7$ für Wasserstoff oder Methyl steht,
$R^8$ für Wasserstoff oder Methyl steht,
$R^9$ für Wasserstoff oder Methyl steht,
M für Wasserstoff, Natrium oder Kalium steht und
W für Halogen oder einen Sulfonyloxy-Rest, wie vorzugsweise Chlor, Brom, Iod, $-OSO_2OCH_3$, $-OSO_2OC_2H_5$, $-OSO_2CH_3$ oder

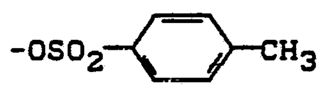

steht.

Bevorzugte erfindungsgemäßen Verbindungen sind außerdem Additionsprodukte, aus Salzen von Metallen der II. bis IV. Haupt-und der I. und II. sowie IV. bis VIII. Nebengruppe und denjenigen substituierten N-(2-Cyan-2-oximinoacetyl)-aminonitrilen der allgemeinen Formel (I), in denen die Substituenten $R^1$, $R^2$ und A die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen diese Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise Methylaminoacetonitril-hydrochlorid und (E)-2-Cyan-2-methoximinoacetylchlorid als Ausgangsstoffe, so kann der Ablauf des erfindungsgemäßen Verfahrens (a) durch das folgende Formelschema wiedergegeben werden:

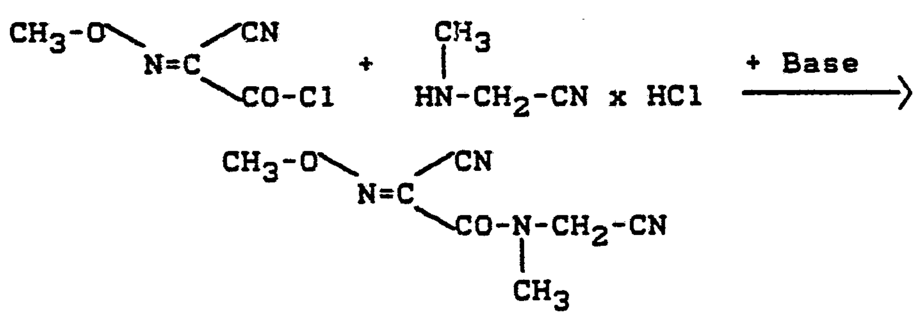

Die für die Durchführung des erfindungsgemäßen Verfahrens (a) als Ausgangsstoffe zu verwendenden (E)-2-Cyan-2-oximinoessigsäuren der allgemeinen Formel (II) sind teilweise bekannt (vgl. z.B. I. J. Belasco und F. J. Baude, Pestic. Sci., 12 , 27 (1981)) bzw. können sie nach allgemein bekannten Verfahren erhalten werden, wie beispielsweise, wenn man die Carbonsäureester der allgemeinen Formel (IX)

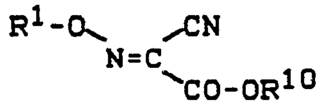

(IX)

in welcher
$R^1$ die oben angegebene Bedeutung hat und
$R^{10}$ für Methyl oder Ethyl steht,
zunächst mit einem Alkalihydroxid, wie beispielsweise Natriumhydroxid, und anschließend mit einer Säure, wie beispielsweise Salzsäure oder Schwefelsäure, oder mit einem sauren Ionenaustauscher in Gegenwart eines Verdünnungsmittel, wie beispielsweise Wasser, eines Alkohols, Ether oder eines Gemisches von Alkoholen oder Ethern mit Wasser, bei Temperaturen zwischen 0 °C und 80 °C, vorzugsweise zwischen 20 °C und 40 °C, umsetzt.

Vorzugsweise erfolgt die Verseifung mit Kaliumhydroxid in Gegenwart eines Wasser/Ethanol-Gemisches, wobei das Reaktionsgemisch bei 20 °C im Vakuum eingedampft wird und das entstehende Kaliumsalz mit
z.B. Oxalylchlorid zum entsprechenden Säurechlorid umgesetzt wird.

Die Carbonsäureester der allgemeinen Formel (IX) sind teilweise bekannt (vgl. I.J. Belasco und F.J. Baude, Pestic, Scie., 12, 27, 1981) oder können nach dem dort beschrieben Verfahren erhalten werden, in dem man die E-Isomeren der Carbonsäureester der allgemeinen Formel (X)

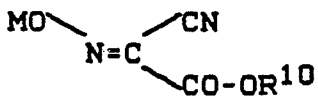

(X)

in welcher
$R^{10}$ die oben genannte Bedeutung hat und
M für Wasserstoff, Natrium oder Kalium, vorzugsweise für Natrium steht,
mit einer Verbindung der allgemeinen Formel (XI)
$R^1$-W (XI)
in welcher
$R^1$ die oben angegebene Bedeutung hat und
W für Halogen oder einen Sulfonyloxy-Rest, wie vorzugsweise Chlor, Brom, Iod, $-OSO_2OCH_3$, $-OSO_2OC_2H_5$, $-OSO_2CH_3$ oder

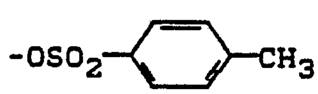

steht,
gegebenenfalls in Gegenwart einer Base, wie beispielsweise Kaliumcarbonat, Triethylamin oder Diazabicycloundecen (DBU), und in Gegenwart eines Lösungsmittels, wie beispielsweise Aceton, Dimethylformamid, Dimethylsulfoxid oder Acetonitril, bei Temperaturen zwischen 0 °C und 120 °C umsetzt.

Die E-Isomeren der Carbonsäureester der allgemeinen Formel (X) sind bekannt (vergl. z. B. G. Kinast, Liebigs Ann. Chem, 1981, 1561; M. Conrad und A. Schulze, Ber. dtsch. chem. Ges., 42, 735, 1909) oder können nach den dort beschriebenen Verfahren erhalten werden.

Die Verbindungen der allgemeinen Formel (XI) sind allgemein bekannte Alkylierungsmittel.

Als carboxy-aktivierte Derivate der Carbonsäuren der allgemeinen Formel (II) kommen alle carboxy-aktivierten Derivate in Frage, wie Alkylester, Säuerhalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxysuccinimidester sowie mit Kondensationsmitteln, wie z.B. Dicyclohexylcarbodiimid oder Carbonyldiimidazol, in situ erzeugte aktivierte Derivate der Carbonsäuren.

Vorzugsweise werden die den Carbonsäuren der allgemeinen Formel (II) entsprechenden Säurechloride eingesetzt. Sie können hergestellt werden, indem man die Carbonsäuren der allgemeinen Formel (II) oder deren Salze mit einem Halogenierungsmittel, wie beispielsweise Phosphorpentachlorid, Thionylchlorid, Oxalylchlorid oder Phosgen, in allgemein bekannter Art und Weise umsetzt. Bevorzugt ist der Einsatz von Oxalylchlorid zusammen mit dem Natrium-oder Kaliumsalz der Carbonsäure der allgemeinen Formel (II).

Die Acylierung mit den carboxy-aktivierten Derivaten kann in wäßrigem oder nichtwäßrigem Medium durchgeführt werden; geeignete Medien sind dabei Ketone, wie z.B. Aceton; Ester, wie z.B. Ethylacetat; Amide, wie z.B. Dimethylformamid; Nitrile, wie z.B. Acetonitril; Chlorkohlenwasserstoffe; wie z.B. Methylenchlorid; Kohlenwasserstoffe, wie z.B. Toluol; oder Ether, wie z.B. Tetrahydrofuran bzw. deren Mischungen.

Die Acylierung mit einem Säurehalogenid kann in Gegenwart eines Säurebindemittels, wie vorzugsweise eines tertiären Amins, wie z.B. Triethylamin, Pyridin, oder einer anorganischen Base, wie z.B. Natriumcarbonat oder Calciumcarbonat, durchgeführt werden.

Die für die Durchführung der erfindungsgemäßen Verfahren (a) und (c) zu verwendenden Amine der allgemeinen Formel (III) sind bekannt bzw. können nach allgemein bekannten Verfahren in einfacher analoger Weise hergestellt werden (vergl. A. Zobacova in "Methodicum Chimicum", Band 6, S. 662, G. Thieme Verlag, Stuttgart 1976).

Der Ablauf des erfindungsgemäßen Verfahrens b) kann beispielsweise durch das folgende Formelschema wiedergegeben werden.

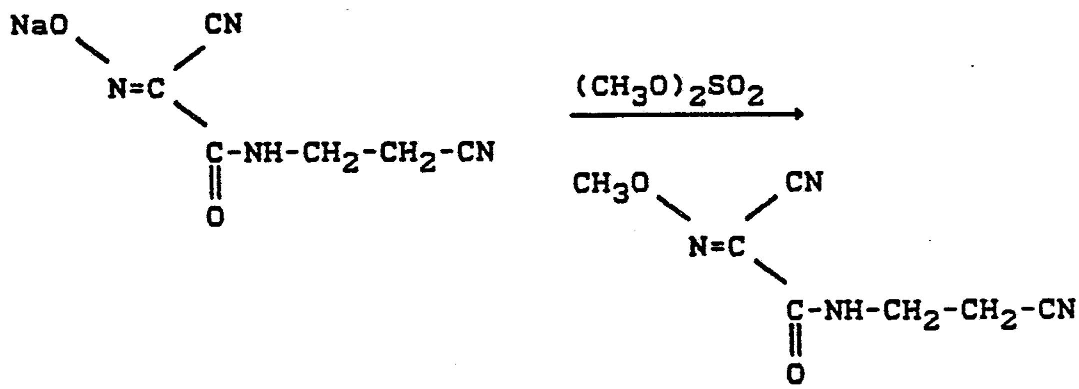


Die für die Durchführung des erfindungsgemäßen Verfahrens (b) als Ausgangsstoffe zu verwendenden E-Oxime der allgemeinen Formel (IV) sind noch nicht bekannt.

Man erhält sie beispielsweise, wenn man Verbindungen der allgemeinen Formel (XII)

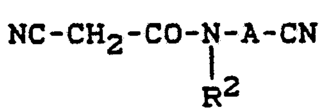
(XII)

in welcher

$R^2$ und A die oben angegebenen Bedeutungen haben,

mit salpetriger Säure oder Derivaten der salpetrigen Säure gegebenenfalls in Gegenwart eines Verdünnungsmittels und gegebenenfalls in Gegenwart einer Säure oder einer Base umsetzt.

Als Verdünnungsmittel kommen für das Verfahren zur Herstellung von Verbindungen der allgemeinen Formel (IV) Wasser und organische Lösungsmittel in Frage. Hierzu gehören vorzugsweise Alkohole, wie Methanol oder Ethanol, Carbonsäuren, wie Eisessig sowie Ether, wie Dioxan oder Tetrahydrofuran.

Das obengenannte Verfahren kann gegebenenfalls im Zweiphasensystem mit Wasser und einem mit Wasser nicht mischbaren Lösungsmittel, wie z. B. Toluol oder Methylenchlorid, durchgeführt werden.

Das Verfahren zur Herstellung von Verbindungen der allgemeinen Formel (IV) wird mit salpetriger Säure oder Derivaten der salpetrigen Säure durchgeführt. Man kann alle üblicherweise verwendbaren Derivate der salpetrigen Säure einsetzen. Vorzugsweise arbeitet man mit Nitrosylchlorid, Salpetrigsäureanhydrid oder Alkylnitriten, wie Ethylnitrit, tert.-Butylnitrit sowie insbesondere Isoamylnitrit. Die salpetrige Säure wird "in situ" aus einem Alkalinitrit, wie insbesondere dem Natriumnitrit und einer Säure, wie insbesondere der Chlorwasserstoffsäure oder Essigsäure hergestellt.

Das obengenannte Verfahren zur Herstellung von Verbindungen der allgemeinen Formel (IV) wird gegebenenfalls in Gegenwart einer Säure oder Base durchgeführt.

Als Säuren kommen alle üblicherweise verwendbaren organischen und anorganischen Säuren in Frage. Hierzu gehören vorzugsweise die Chlorwasserstoffsäure und Essigsäure.

Als Basen kommen alle üblicherweise verwendbaren starken anorganischen und organischen Basen in Frage. Hierzu gehören vorzugsweise Alkalimetallalkoholate, wie Natriummethylat und Natriumethylat, sowie Alkaliamide, wie Natriumamid.

Die Reaktionstemperaturen können bei der Durchführung des Verfahrens zur Herstellung von Verbindungen der allgemeinen Formel (IV) in einem größeren Bereich variiert werden. Im allgemeinen arbeitet man bei Temperaturen zwischen -20 °C und 120 °C, vorzugsweise bei Temperaturen zwischen 0 °C und 40 °C.

Bei der Durchführung des Verfahrens zur Herstellung von Verbindungen der allgemeinen Formel (IV) setzt man auf 1 Mol der Verbindung der allgemeinen Formel (XII) im allgemeinen 1 bis 5 Mol, vorzugsweise 1 bis 3 Mol salpetrige Säure bzw. eines Derivates der salpetrigen Säure und gegebenenfalls 1 bis 5 Mol, vorzugsweise 1 bis 3 Mol Säure oder gegebenenfalls 1 bis 5 Mol, vorzugsweise 1 bis 3 Mol Base ein.

Die Aufarbeitung und Isolierung der Reaktionsprodukte der allgemeinen Formel (IV) erfolgt in üblicher Art und Weise, z. B. durch Abfiltrieren von kristallinen Produkten oder durch Extraktion mit einem geeigneten organischen Lösungsmittel.

Die Verbindungen der allgemeinen Formel (XII) sind neu. Sie können in allgemein bekannter Art und Weise erhalten werden, indem man z. B.

α) Cyanessigester der allgemeinen Formel (XIII)

$$NC-CH_2-\overset{\overset{O}{\|}}{C}-OR^{10} \quad (XIII)$$

in welcher

$R^{10}$ für Methyl oder Ethyl steht,

mit Aminonitrilen der allgemeinen Formel (III)

$$\underset{\displaystyle R^2}{\underset{|}{HN}}-A-CN \qquad (III)$$

in welcher

$R^2$ und A die oben angegebenen Bedeutungen haben,

gegebenenfalls in einem Lösungsmittel, wie beispielsweise Methanol, Ethanol, Diethylether, 1,2-Dimethoxyethan, Dimethylformamid oder deren Mischungen mit Wasser bei 0 °C bis 60 °C, vorzugsweise bei Raumtemperatur, umsetzt;

oder

β) ein aktiviertes Derivat der Cyanessigsäure der allgemeinen Formel (XIV)

$NC-CH_2-CO-OH$ (XIV)

mit Aminonitrilen der allgemeinen Formel (III)

$$\underset{\displaystyle R^2}{\underset{|}{HN}}-A-CN \qquad (III)$$

in welcher

$R^2$ und A die oben angegebenen Bedeutungen haben,

in Gegenwart eines organischen Lösungsmittels, wie beispielsweise Tetrahydrofuran oder Dimethylformamid und gegebenenfalls in Gegenwart eines Säurebindemittels, wie beispielsweise Pyridin oder Triethylamin, bei Temperaturen zwischen -40 °C und +40 °C umsetzt.

Als aktivierte Derivate der Cyanessigsäure werden vorzugsweise das Säurechlorid, aktivierte Ester und gemischte Anhydride eingesetzt,
oder
γ) Halogenacetamide der allgemeinen Formel (XV)

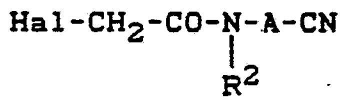
(XV)

in welcher
$R^2$ und A die oben angegebenen Bedeutungen haben und
Hal für ein Halogenatom, wie Chlor oder Brom steht,
mit Alkalicyaniden der allgemeinen Formel (XVI)
MCN (XVI)
in welcher
M für ein Alkalimetallatom, wie Natrium oder Kalium steht,
in Gegenwart eines Lösungsmittels, wie beispielsweise Wasser, Dimethylsulfoxid, Dimethylformamid, N-Methylpyrrolidon, Acetonitril oder Tetrahydrofuran und gegebenenfalls in Gegenwart eines Phasentransferkatalysators, wie z. B. 18-Krone-6, bei Temperaturen zwischen 0 °C und 200 °C umsetzt.

Die Verbindungen der allgemeinen Formeln (XIII), (XIV), (XV) und (XVI) sind bekannt bzw. können in allgemein bekannter Art und Weise erhalten werden.

Die für die Durchführung des erfindungsgemäßen Verfahrens (b) als Ausgangsstoffe zu verwendenden Verbindungen der allgemeinen Formel (V) sind allgemein bekannte Verbindungen der organischen Chemie.

Der Ablauf des erfindungsgemäßen Verfahrens c) kann beispielsweise durch das folgende Formelschema wiedergegeben werden:

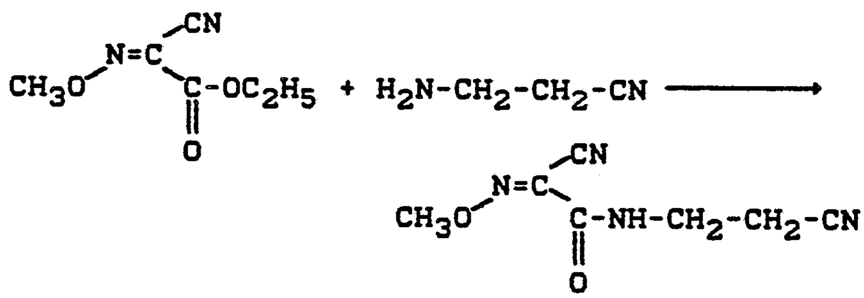

Die für die Durchführung des erfindungsgemäßen Verfahrens (c) als Ausgangsstoffe zu verwendenden (Z)-2-Cyan-2-oximino-acetylchloride der allgemeinen Formel (VI) sind noch nicht bekannt. Die entsprechenden Alkylester sind teilweise bekannt (vgl. z.B. J. Antibiot. 37, 557 (1984)).

Man erhält die Säurechloride beispielsweise, wenn man Verbindungen der allgemeinen Formel (XVII)

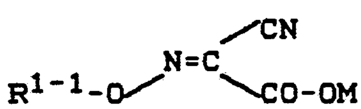
(XVII)

in welcher
$R^{1-1}$ die oben angegebene Bedeutung hat und
M für Wasserstoff oder ein Alkalimetallatom, wie beispielsweise Natrium oder Kalium steht,
mit einem Halogenierungsmittel gegebenenfalls in Gegenwart eines Verdünnungsmittels und gegebenenfalls in Gegenwart eines Katalysators umsetzt.

Als Verdünnungsmittel kommen für das obengenannte Verfahren zur Herstellung von Verbindungen der allgemeinen Formel (VI) inerte organische Lösungsmittel in Frage. Hierzu gehören vorzugsweise Ether, wie Diethylether; chlorierte Kohlenwasserstoffe, wie Methylenchlorid; Ester, wie Essigester; Nitrile, wie Acetonitril; sowie Kohlenwasserstoffe, wie Toluol.

Das Verfahren zur Herstellung der Chloride der allgemeinen Formel (VI) wird mit Halogenierungsmittel durchgeführt. Man kann alle üblicherweise verwendbaren Halogenierungsmittel einsetzen. Vorzugsweise arbeitet man mit Oxalylchlorid, Phosphorpentachlorid, Thionylchlorid oder Phosgen. Dabei ist darauf zu achten, daß die Halogenierungsmittel keinen freien Chlorwasserstoff enthalten, da sonst während des Verfahrens Isomerisierung zum E-Isomeren erfolgen kann.

Das Verfahren zur Herstellung von Verbindungen der allgemeinen Formel (VI) wird gegebenenfalls in Gegenwart eines Katalysators durchgeführt. Hierzu gehören vorzugsweise Dimethylformamid und Triphenylphosphin.

Die Reaktionstemperaturen können bei der Durchführung des Verfahrens zur Herstellung von Verbindungen der allgemei nen Formel (VI) in einem größeren Bereich variiert werden. Im allgemeinen arbeitet man bei Temperaturen zwischen -20 °C und 80 °C, vorzugsweise bei Temperaturen zwischen -10 °C und 30 °C, insbesondere bei 0 °C.

Bei der Durchführung des Verfahrens zur Herstellung der Chloride der allgemeinen Formel (VI) setzt man auf 1 Mol der Verbindung der allgemeinen Formel (II) im allgemeinen 1 bis 10 Mol, vorzugsweise 1 bis 3 Mol Halogenierungsmittel ein.

Zur Isolierung der Verbindung der allgemeinen Formel (VI) wird das Reaktionsgemisch in üblicher Art und Weise aufgearbeitet.

Die Verbindungen der allgemeinen Formel (XVII) sind bisher noch nicht bekannt. Sie lassen sich jedoch nach im Prinzip bekannten Verfahren in einfacher Weise herstellen. So erhält man beispielsweise die Verbindungen der allgemeinen Formel (XVII), indem man die Carbonsäureester der allgemeinen Formel (XVIII)

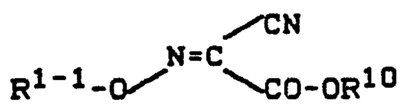

(XVIII)

in welcher
$R^{1-1}$ die oben angegebene Bedeutung hat und
$R^{10}$ für Methyl oder Ethyl steht,
in einem Lösungsmittel, wie beispielsweise Wasser, Methanol oder Ethanol mit einem Alkalihydroxid, wie beispielsweise Kalium-oder Natriumhydroxid bei 0 °C bis 40 °C verseift und das Alkalisalz durch z. B. vorsichtiges Eindampfen der Reaktionslösung isoliert.

Die Carbonsäureester der allgemeinen Formel (XVIII) sind teilweise bekannt (vergl. z. B. J. Goto, K. Sakane und T. Teraji, J. Antibiot. 37, 557 (1984)).

Sie können erhalten werden, indem man Amide der allgemeinen Formel (XIX)

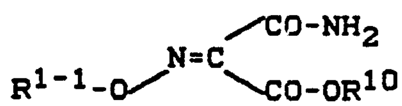

(XIX)

in welcher
$R^{1-1}$ und $R^{10}$ die oben angegebene Bedeutung haben,
mit einem Dehydratisierungsmittel, wie beispielsweise Trifluoressigsäureanhydrid, Trichloracetylchlorid, Mesylchlorid, Tosylchlorid, Titantetrachlorid oder N,N-Dimethylchlorformimidiumchlorid, in Gegenwart einer tertiären Base wie beispielsweise Pyridin, Triethylamin oder N-Methylmorpholin, und gegebenenfalls in Gegenwart eines Lösungsmittels, wie beispielsweise Dioxan, Tetrahydrofuran, Acetonitril, Methylenchlorid oder Pyridin bei Temperaturen zwischen -20 °C und 40 °C dehydratisiert.

Die Amide der allgemeinen Formel (XIX) sind teilweise bekannt (vergl. z. B. J. Goto, K. Sakane und T. Teraji, J. Antibiot. 37, 557 (1984)).

Sie können erhälten werden, indem man Verbindungen der allgemeinen Formel (XX)

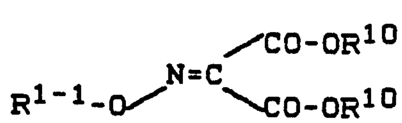

(XX)

in welcher
$R^{1-1}$ und $R^{10}$ die oben angegebene Bedeutung haben,

mit Ammoniak in Gegenwart eines Lösungsmittels, wie beispielsweise Methanol, Ethanol, Diethylether, Dimethoxyethan, Dimethylformamid oder Acetonitril bei Temperaturen zwischen -20 °C und 30 °C umsetzt.

Die Verbindungen der allgemeinen Formel (XX) sind teilweise bekannt (vergl. z. B. J. Goto, K. Sakane und T. Teraji, J. Antibiot. 37, 557 (1984)).

Sie können erhalten werden, indem man Verbindungen der allgemeinen Formel (XXI)

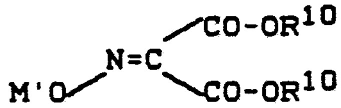
(XXI)

in welcher
$R^{10}$ die oben angegebene Bedeutung hat, und
M' für Wasserstoff, Natrium oder Kalium steht,
mit Verbindungen der allgemeinen Formel (XXII)
$R^{1-1}$-W (XXII)
in welcher
$R^{1-1}$ die oben angegebene Bedeutung hat und
W für Halogen oder einen Sulfonyl(di)oxy-Rest, wie vorzugsweise Chor, Brom, Iod, $-OSO_2OCH_3$, $-OSO_2OC_2H_5$, $-OSO_2CH_3$

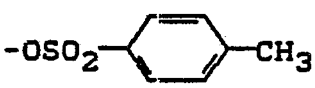

oder steht,
gegebenenfalls in Gegenwart einer Base, wie beispielsweise Kaliumcarbonat, Triethylamin oder Diazabicycloundecen (DBU), und in Gegenwart eines Lösungsmittels, wie beispielsweise Aceton, Dimethylformamid, Dimethylsulfoxid oder Acetonitril, bei Temperaturen zwischen 0 °C und 120 °C umsetzt.

Die Verbindungen der allgemeinen Formeln (XXI) und (XXII) sind allgemein bekannte Verbindungen der organischen Chemie.

Die für die Durchführung des erfindungsgemäßen Verfahrens (e) als Ausgangsstoffe zu verwendenden Amine der allgemeinen Formel (VII) sind allgemein bekannte Verbindungen der organischen Chemie.

Die für die Durchführung des erfindungsgemäßen Verfahrens (g) als Ausgangsstoffe zu verwendenden Amine der allgemeinen Formel (VIII) sind ebenfalls allgemein bekannte Verbindungen der organischen Chemie.

Die für die Durchführung der erfindungsgemäßen Verfahren (d), (e), (f) und (g) als Ausgangsstoffe zu verwendenden N-[(E)-2-Cyan-2-oximinoacetyl]-aminonitrile der allgemeinen Formeln (I-2), (I-3) und (I-5) sind erfindungsgemäße Verbindungen und können gemäß den erfindungsgemäßen Verfahren (a), (b) oder (c) hergestellt werden.

Der Ablauf der erfindungsgemäßen Verfahren d) bis g) kann beispielsweise durch die folgenden Formelchemata wiedergegeben werden:

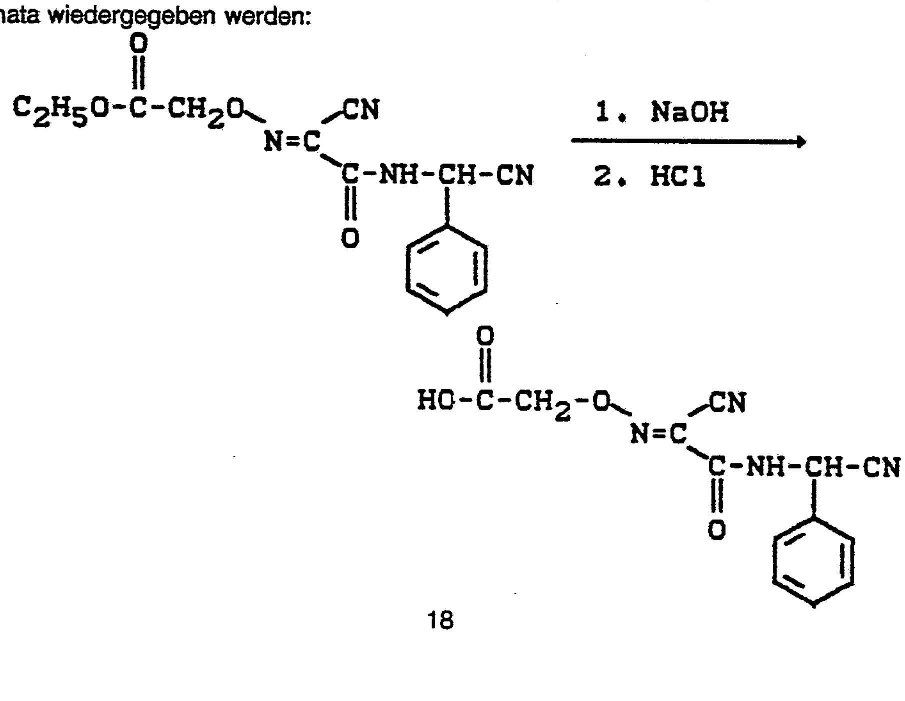

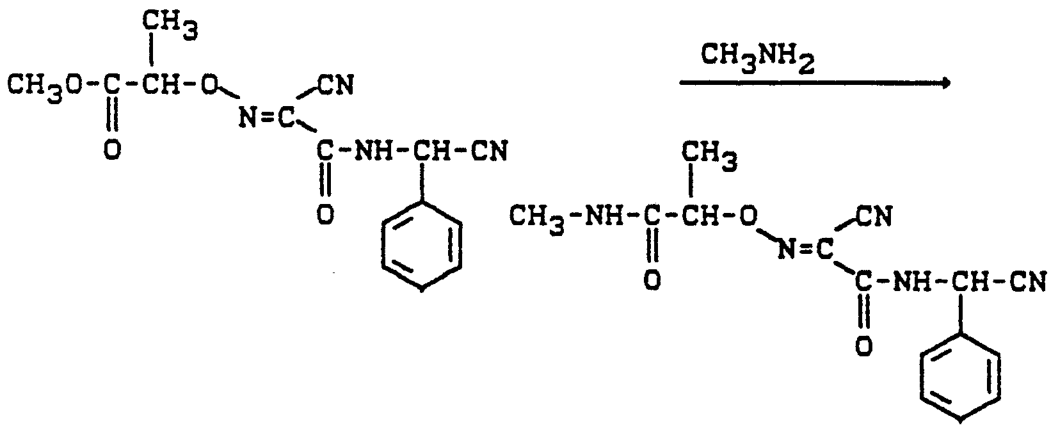


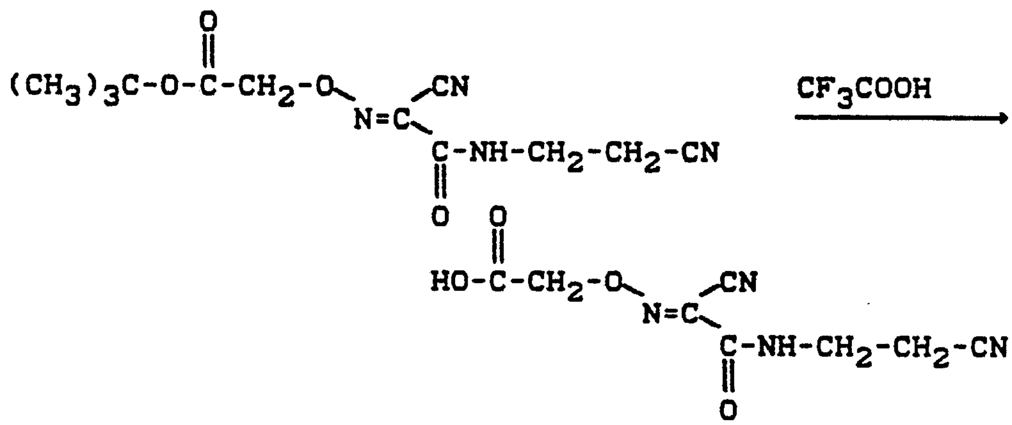


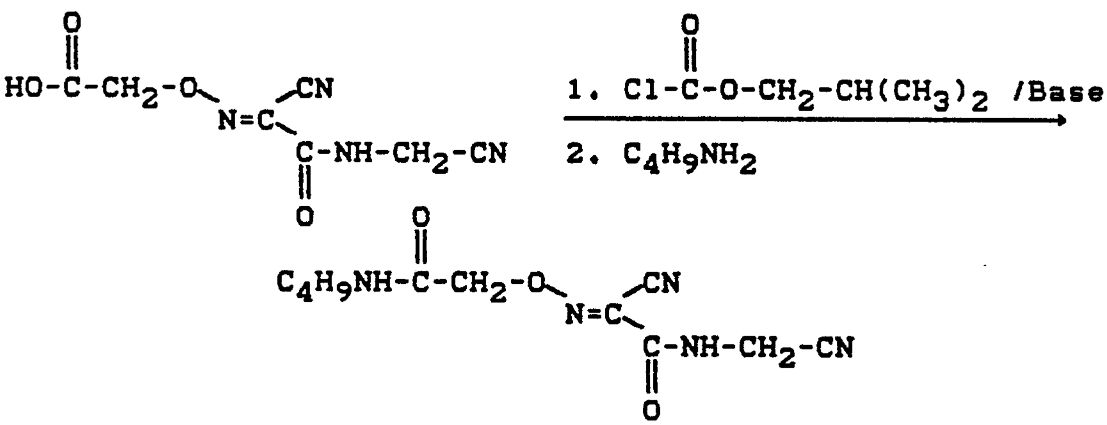


Als Verdünnungsmittel kommen für das erfindungsgemäße Verfahren (a) inerte, organische Lösungsmittel in Frage. Hierzu gehören Ketone, wie Aceton oder Ethylmethylketon; Ester wie Ethyl-oder Methylacetat; Amide wie Dimethylformamid; Nitrile wie Acetonitril; Chlorkohlenwasserstoffe, wie Methylenchlorid oder Chloroform; Kohlenwasserstoffe, wie Toluol oder Ether, wie Tetrahydrofuran bzw. deren Mischungen.

Als Säurebindemittel kommen für das erfindungsgemäße Verfahren (a) übliche anorganische und organische Säurebinder in Frage, Hierzu gehören vorzugsweise tertiäre Amine, wie Triethylamin, Pyridin oder N-Methylmorpholin; sowie anorganische Basen, wie Natriumcarbonat oder Calciumcarbonat.

Das erfindungsgemäße Verfahren (a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung des Verfahrens (a) in einem größeren Bereich variiert werden. Im allgemeinen arbeitet man zwischen -60 °C und +120 °C, vorzugsweise bei -20 °C bis +40 °C.

Bei der Durchführungs des erfindungsgemäßen verfahrens (a) arbeitet man vorzugsweise in äquimolaren Mengen.

Dabei werden die Aminonitrile der allgemeinen Formel (III) als reine Enantiomere (D bzw. L-Form) oder als Racemate eingesetzt.

Als Lösungsmittel kommen für das erfindungsgemäße Verfahren (b) Alkohole, wie Methanol und Ethanol, Ketone, wie Aceton und Methylisobutylketon, Nitrile wie Acetonitril, Dimethylsulfoxid, Dimethylformamid und N-Methylpyrrolidon in Frage.

Als Base kommen für das erfindungsgemäße Verfahren (b) übliche anorganische und organische Basen in Frage. Hierzu gehören vorzugsweise tertiäre Amine, wie Triethylamin, Pyridin und Diazabicycloundecen (DBU); sowie anorganische Basen, wie Natrium-, Kalium-oder Calciumcarbonat.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 120°C.

Bei der Durchführung des erfindungsgemäßen Verfahrens (b) setzt man pro Mol E-Oxim der allgemeinen Formel (IV) im allgemeinen 1 bis 3 Mol, vorzugsweise 1 bis 2 Mol Alkylierungsmittel der allegeminen Formel (V) ein. Die Reaktionsdurchführung, Aufarbeitung und Isolierung der Reaktionsprodukte der allgemeinen Formel (I) erfolgt in allgemein üblicher Weise.

Als Verdünnungsmittel kommen für das erfindungsgemäße Verfahren (c) inerte, organische Lösungsmittel in Frage. Hierzu gehören insbesondere Ketone, wie Aceton oder Ethylmethylketon; Ester wie Ethyl-oder Methylacetat; Amide wie Dimethylformamid; Nitrile wie Acetonitril; Chlorkohlenwasserstoffe, wie Methylenchlorid oder Chloroform; Kohlenwasserstoffe, wie Toluol; Ether, wie Tetrahydrofuran; oder Alkohole, wie Methanol oder i-Propanol, bzw. deren Mischungen.

Als Säurebindemittel kommen für das erfindungsgemäße Verfahren (c) übliche anorganische und organische Säurebinder in Frage. Hierzu gehören vorzugsweise tertiäre Amine, wie Triethylamin, Pyridin oder N-Methylmorpholin; sowie anorganische Basen, wie Natriumcarbonat oder Calciumcarbonat.

Das erfindungsgemäße Verfahren (c) wird gegebenenfalls in Gegenwart eines Katalsators durchgeführt. Beispielsweise genannt seien 4-Dimethylaminopyridin, 1-Hydroxy-benzotriazol oder Dimethylformamid.

Die Temperaturen können bei der Durchführung des Verfahrens (c) in einem größeren Bereich variiert werden. Im allgemeinen arbeitet man zwischen -60 °C und +120 °C, vorzugsweise bei -20 °C bis +40°C.

Bei der Durchführung des erfindungsgemäßen Verfahrens (c) arbeitet man vorzugsweise in äquimolaren Mengen und einem Basenüberschuß von 10 bis 50 Prozent. Es ist darauf zu achten, daß keine freie Chlorwasserstoffsäure entsteht, da sonst Isomerisierung zum E-Isomer erfolgen kann.

Dabei werden die Aminonitrile der allgemeinen Formel (III) als reine Enantiomere (D bzw. L-Form) oder als Racemate eingesetzt.

Die erfindungsgemäße Hydrolyse gemäß Verfahren (d) erfolgt in üblicher Weise mit einem Alkali-oder Erdalkalihydroxid, wie Natriumhydroxid, Kaliumhydroxid und Bariumhydroxid gegebenenfalls in Gegenwart eines wäßrigen oder mit Wasser mischbaren organischen Lösungsmittels. Vorzugsweise verwendet man Wasser, Alkohole, wie Methanol und Ethanol oder Mischungen von Wasser und Alkoholen oder Ethern, wie Dioxan.

Die Temperaturen können bei der Durchführung des erfindungsgemäßen Verfahrens (d) in einem größeren Bereich variiert werden. Im allgemeinen arbeitet man bei Temperaturen zwischen 0 °C und 120 °C, vorzugsweise bei Temperaturen zwischen 20 °C und 80 °C.

Als Lösungsmittel kommen für die Aminolyse gemäß dem erfindungsgemäßen Verfahren (e) alle üblichen mit Wasser mischbaren organischen Lösungsmittel in Frage. Vorzugsweise verwendet man Alkohole, wie Methanol, Mischungen von Alkoholen, wie Methanol und Wasser sowie Glykol.

Die Reaktionstemperaturen können bei der Durchführungs des erfindungsgemäßen Verfahrens (e) in einem größeren Bereich variiert werden. Im allgemeinen arbeitet man bei Temperaturen zwischen -20 °C und 80 °C, vorzugsweise zwischen 0 °C und 60 °C.

Bei der Durchführung des erfindungsgemäßen Verfahren (e) arbeitet man im allgemeinen mit einem großen Überschuß des Amins der allgemeinen Formel (VII), vorzugsweise mit einem dreifachen Überschuß.

Als Lösungsmittel kommen für das erfindungsgemäße Verfahren (f) insbesondere chlorierte Kohlenwasserstoffe, wie beispielsweise Dichlormethan und Chloroform; Ether, wie Dioxan und Tetrahydrofuran; Carbonsäuren, wie Essigsäure; und Ester wie Essigester in Frage.

Als Säure kommen für das erfindungsgemäße Verfahren (f) alle üblichen starken organischen und anorganischen Säuren in Frage, Hierzu gehören insbesondere Chlorwasserstoffsäure, Bromwasserstoffsäure, Ameisensäure und Trifluoressigsäure.

Die Reaktionstemperaturen können bei der Durchführung des erfindungsgemäßen Verfahrens (f) in einem größeren Bereich variiert werden. Im allgemeinen arbeitet man bei Temperaturen zwischen -10 °C und 60 °C, vorzugsweise bei Temperaturen zwischen 10 °C und 30 °C.

Bei der Durchführung des erfindungsgemäßen Verfahrens (f) setzt man pro Mol Verbindung der allgemeinen Formel (I-4) im allgemeinen 1 bis 20 Mol, vorzugsweise 1 bis 10 Mol Säure ein.

Als Verdünnungsmittel kommen für das erfindungsgemäße Verfahren (g) Wasser und organische Lösungsmittel in Frage. Hierzu gehören vorzugsweise Kohlenwasserstoffe, wie Toluol; Chlorkohlenwasserstoffe, wie Dichlormethan; Ester, wie Essigester; Ether, wie Dioxan, Tetrahydrofuran und Dimethoxyethan; Nitrile, wie Acetonitril; Amide, wie Dimethylformamid.

Das erfindungsgemäße Verfahren (g) wird gegebenenfalls in Gegenwart einer Base durchgeführt. Hierzu gehören alle üblicherweise verwendbaren anorganischen und organischen Basen, wie insbesondere Kaliumcarbonat, Triethylamin, N-Methylmorpholin und Pyridin.

Das erfindungsgemäße Verfahren (g) wird gegebenenfalls in Gegenwart eines Katalysators durchgeführt. Beispielsweise genannt seinen Dimethylformamid, 4-Dimethylaminopyridin und 1-Hydroxybenzotriazol.

Die Reaktionstemperaturen können bei der Durchführung des erfindungsgemäßen Verfahrens (g) in einem größeren Bereich variiert werden. Im allgemeinen arbeitet man bei Temperaturen zwischen -60 °C und 40 °C, vorzugsweise bei Temperaturen zwischen -20 °C und 20 °C.

Bei der Durchführung des erfindungsgemäßen Verfahrens (g) setzt man pro Mol des carboxy-aktivierten Derivates der Carbonsäure der allgemeinen Formel (I-2) im allgemeinen 1 bis 2 Mol, vorzugsweise 1 Mol Amin der allgemeinen Formel (VIII) und im allgemeinen 1 bis 2 Mol Base ein.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B. Ethanol und Hinzufügen zu Verbindungen der allgemeinen Formel (I). Man kann Metallsalz-Komplexe in bekannter Weise, z.B. durch Abfiltrieren, isolieren und gegenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt.

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis; Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea;
(Konidienform: Drechslera, Syn: Helminthosporium);
Cochliobolus-Arten, wie beispielsweise Cochliobolus sativus;
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;

Leptosphaeria-Arden, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz-und Saatgut, und des Bodens.

Als Schädlingsbekämpfungsmittel können die erfindungsgemäßen Wirkstoffe mit besonders gutem Erfolg zur Bekämpfung von Phytophthora-Arten, wie beispielsweise Phytophthora infestans, an Tomaten eingesetzt werden.

Besonders hervorzuheben ist, daß die erfindungsgemäßen Wirkstoffe nicht nur eine protektive Wirkung entfalten, sondern auch kurativ wirksam sind, also bei Anwendung nach der Kontamination mit den Sporen des Pilzes.

Ferner eignen sich die erfindungsgemäßen Wirkstoffe sehr gut zur Bekämpfung von Pyricularia-Arten wie Pyricularia oryzae am Reis und zeigen auch systemische Wirkung gegen Peronospora an Erbsen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lössungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder -schaumerzeugende Mittel kommen in Frage: z.B. nichtionogene und anionische Emulgatoren, wie PolyoxyethylenFettsäure-Ester, Polyoxyethylen-Fettalkohol-Ether, z.B. Alkylarylpolyglykol-Ester, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

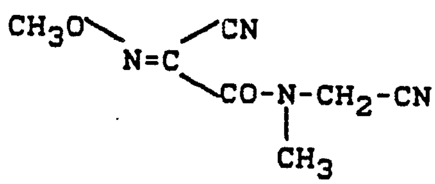

Zu einer Lösung von 5,35 g (0,05 Mol) Methylaminoacetonitril-hydrochlorid in 70 ml Dimethylformamid gibt man 10,1 g (0,1 Mol) Triethylamin und bei 0 °C tropfenweise 7,35 g (0,05 Mol) (E)-2-Cyan-2-methoximino-acetylchlorid. Man rührt 1 Stunde bei 0 °C und 18 Stunden bei Raumtemperatur. Das Reaktionsgemisch wird in 100 ml Wasser gegossen, mit Essigester (dreimal je 50 ml) extrahiert, der Extrakt mit je 60 ml 1M Salzsäure, NatriumhydrogencarbonatLösung und Kochsalz-Lösung gewaschen, über Natriumsulfat getrocknet und im Vakuum eingedampft. Der Rückstand wird aus Essigester/Petrolether umkristallisiert.

Man erhält 5,7 g N-[(E)-2-Cyan-2-methoximino-acetyl]-N-methylaminoacetonitril vom Schmelzpunkt 91 °C - 92 °C.

Herstellung des Ausgangsproduktes

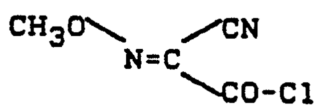

20 g (0,12 Mol) des Kaliumsalzes der (E)-2-Cyan-2-methox iminoessigsäure werden in 250 ml trockenem Ether suspendiert und nach Zugabe von einigen Tropfen Dimethylformamid bei 0 °C tropfenweise mit 76,2 g (0,6 Mol) Oxalylchlorid versetzt. Das Reaktionsgemisch wird 2 Stunden bei 0 °C gerührt, filtriert, das Filtrat im Vakuum bei Raumtemperatur mit Methylenchlorid vom restlichen Oxalylchlorid befreit.

Man erhält 13,5 (77 % der Theorie) (E)-2-Cyan-2-methoximinoacetylchlorid als gelbes Öl, das sofort weiter umgesetzt wird.

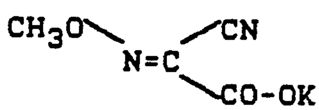

In eine Lösung von 124,8 g (0,672 Mol) 84-prozentigem (E)-2-Cyan-2-methoximino-essigsäureethylester in 500 ml Ethanol wird bei 20 °C eine Lösung von 45,1 g (0,806 Mol) Kaliumhydroxid in 500 ml Wasser getropft und das Reaktionsgemisch eine Stunde bei 40 °C gehührt. Die Lösung wird im Vakuum bei 40 °C eingedampft, der Rückstand mit Methanol 30 Minuten verrührt, abgesaugt, mit Ethanol, Acetonitril und Dichlormethan gewaschen und bei Raumtemperatur getrocknet.

Man erhält 58,6 g (53 % der Theorie) des Kaliumsalzes der (E)-2-Cyan-2-methoximino-essigsäure.

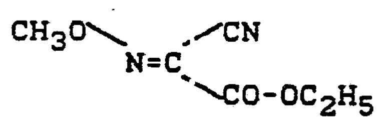

In eine Suspension von 164 g (1 Mol) (E)-2-Cyan-2-hydroximinoessigsäureethylester, Natriumsalz (G. Kinast, Liebigs Ann. Chem., 1981, 1561) und 138 g pulverisiertem Kaliumcarbonat in 1,5 1 Aceton werden 161 g (1,25 Mol) Dimethylsulfat (98 %ig) in 30 Minuten getropft und das Reaktionsgemisch 3 Stunden unter Rückfluß erhitzt. Nach Abkühlen wird über Kieselgur filtriert und eingedampft.

Man erhält 124,8 g (68 % der Theorie) (E)-2-Cyan-2-methoximino-essigsäure-ethylester als rotbraunes Öl von 85 % Reinheit (GC). Nach Chromatographie an der fünffachen Menge Kieselgel 60 mit Chloroform erhält man ein 93-prozentiges hellgelbes Öl.

Beispiel 2

(Verfahren C)

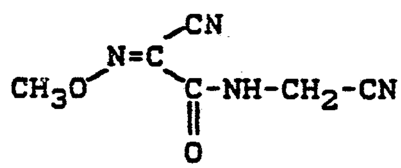

Zu einer Lösung von 22 g (0,238 Mol) Aminoacetonitrilhydrochlorid, 48,3 g (0,477 Mol) Triethylamin und 2,9 g (0,024 Mol) 4-Dimethylaminopyridin in 150 ml trockenem Methylenchlorid tropft man bei 0° C eine Lösung von (Z)-2-Cyan-2-methozimino-acetylchlorid in 30 ml trockenem Methylenchlorid zu und rührt 18 Stunden bei Raumtemperatur. Nach Verdünnen mit 100 ml Methylenchlorid wird mit 1M Salzsäure, 10 %iger Natriumhydrogencarbonatlösung und ge sättigter Kochsalzlösung gewaschen, getrocknet und eingedampft. Der Rückstand wird in Essigester aufgenommen, über Kieselgel filtriert, das Filtrat mit Aktivkohle behandelt, filtriert und eingedampft. Der Rückstand kristallisiert bei Behandlung mit Isopropanol und Petrolether. Man erhält 1 g (2,5 % der Theorie) N-[(Z)-2-Cyan-2-methoximinoacetyl]-aminoacetonitril vom Schmelzpunkt 74 - 75°C. Die Konfiguration ist durch $^{13}C$-Kernresonanzspektroskopie gesichert.

Beispiel 3

(Verfahren c)

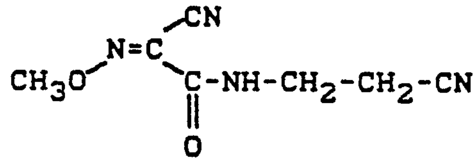

Eine Lösung von 11 g (0,07 Mol) (Z)-2-Cyan-2-methoximinoessigsäureethylester und 5 g (0,07 Mol) 3-Aminopropionitril in 50 ml Methanol wird 1 Stunde bei Raumtemperatur gerührt. Der nach dem Eindampfen verbleibende Rückstand wird aus Essigester/Petrolether umkristallisiert. Man erhält 10,9 g (86,5 % der Theorie) N-[(Z)-2-Cyan-2-methoximino-acetyl]-3-aminopropionitril vom Schmelzpunkt 72° C. Die Z-Konfiguration ist durch $^{13}C$-Kernresonanzspektroskopie gesichert.

In analoger Weise und entsprechend den erfindungsgemäßen Verfahren werden die nachfolgenden N-(2-Cyan-oximinoacetyl)-aminonitrile der Formel (I) erhalten

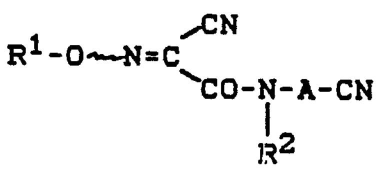 (I)

| Beisp.-Nr. | $R^1$ | $R^2$ | A | Physikalische Daten |
|---|---|---|---|---|
| 4 | 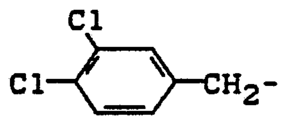 | $CH_3$- | -$CH_2$- | Öl (E-Isomer) |
| 5 | 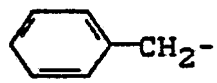 | $CH_3$- | -$CH_2$- | Öl (E-Isomer) |
| 6 | $CH_3$- | H | -$CH_2$- | Fp: 81-83° C (E-Isomer) |
| 7 | $CH_3$- | H | 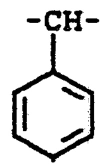 (R,S) | Fp: 84-85° C (E-Isomer) |
| 8 | $CH_3$- | H | -$CH_2$-$CH_2$- | Fp: 75-76° C (E-Isomer) |
| 9 | $CH_3$- | H | 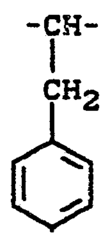 (R,S) | Fp: 88-90° C (E-Isomer) |
| 10 | $CH_3$- | H | 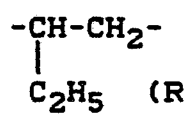 (R,S) | Fp: 51-53° C (E-Isomer) |
| 11 | 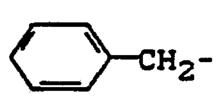 | H | -$CH_2$- | Fp:109-110° C (E-Isomer) |
| 12 | 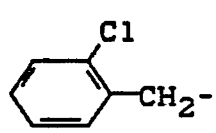 | $CH_3$- | -$CH_2$- | Öl (E-Isomer) |

| Beisp.-Nr. | $R^1$ | $R^2$ | A | Physikalische Daten |
|---|---|---|---|---|
| 13 | $CH_3$- | H | >CH-$C_3H_7$-n (R,S) | Öl (E-Isomeres) |
| 14 | $CH_3$- | H | >CH-$C_3H_7$-i (R,S) | Fp: 90° C (E-Isomeres) |
| 15 | $CH_3$- | H | >CH-$CH_3$ (R,S) | Öl (E-Isomeres) |
| 16 | $CH_3$- | H | >CH-$C_2H_5$ (R,S) | Öl (E-Isomeres) |
| 17 | $CH_3$- | H | >CH-$(CH_2)_5CH_3$ (R,S) | Öl (E-Isomeres) |
| 18 | $CH_3$- | H | >CH-$CH_2CH_2$- 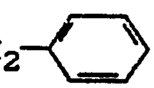 | Öl (E-Isomeres) |
| 19 | $CH_3$- | H | 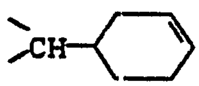 | Öl (E-Isomeres) |

Herstellung von Zwischenprodukten gemäß Formel (IV)

Beispiel (IV-1)

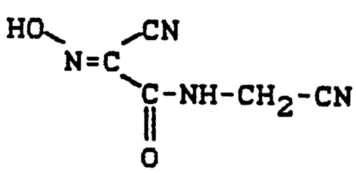

(IV-1)

In eine Suspension von 2,5 g (0,02 Mol) N-Cyanacetylaminoacetonitril und 2,4 g (0,02 Mol) Isoamylnitrit in 10 ml absolutem Ethanol tropft man bei 20° C (Kühlung) 10 ml 2M Natriumethylatlösung und rührt 15 Stunden bei Raumtemperatur. Die Reaktionslösung wird vorsichtig mit 1 g (0,01 Mol) konzentrierter Schwefelsäure versetzt und im Vakuum bei Raumtemperatur eingedampft. Der Rückstand wird mit 20 ml Wasser aufgenommen und dreimal mit je 30 ml Essigester extrahiert. Nach dem Trocknen und Behandeln mit Aktivkohle wird der Extrakt eingedampft. Der Rückstand kristallisiert bei Behandlung mit Petrolether. Man erhält 1,9 g (62,5 % der Theorie) N-[(E)-2-Cyan-2-hydroximinoacetyl]-aminoacetonitril vom Schmelzpunkt 140 - 145° C.

Herstellung der Ausgangsverbindung:

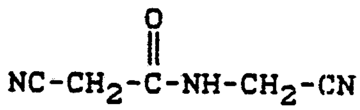 (XII-1)

Eine Lösung von 0,5 Mol Aminoacetonitril in 750 ml Wasser (aus 46,3 g (0,5 Mol) Aminoacetonitril-hydrochlorid mit dem stark basischen Anionenaustauscher Lewatit MP 500 freigesetzt) versetzt man mit 49,5 g (0,5 Mol) Cyanessigsäuremethylester und läßt die Mischung 15 Stunden bei Raumtemperatur stehen. Man wäscht mit Ether, extrahiert fünfmal mit Essigester und dampft den Essigesterextrakt ein. Der Rückstand wird über 150 g Kieselgel (Glasnutsche 12 cm Ø) gereinigt: Elution mit Methylenchlorid und anschließend Essigester. Nach dem Eindampfen des Essigestereluats erhält man 16,3 g (26,5 % der Theorie) N-Cyan-acetyl-aminoacetonitril vom Schmelzpunkt 87-90°C.

In analoger Weise und entsprechend den erfindungsgemäßen Verfahren werden die nachfolgenden Verbindungen erhalten:

(IV-2) 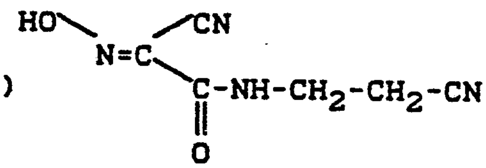 Fp: 146-48° C (E-Isomeres)

(XII-2) 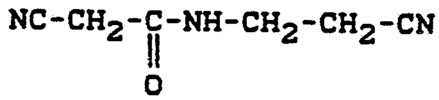 Fp: 75° C

<u>Verwendungsbeispiele</u>

In den nachfolgenden Verwendungsbeispielen wird die nachfolgend angegebene Substanz als Vergleichsverbindung eingesetzt:

(A) 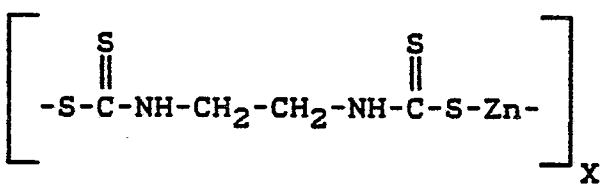

<u>Beispiel A</u>

Phytophthora-Test (Tomate) /kurativ

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele:
1, 4, 5, 6, 8 und 11.

## Ansprüche

1. N-(2-Cyan-2-oximinoacetyl)-aminonitrile der allgemeinen Formel (I)

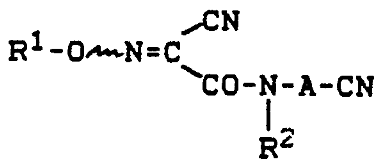
(I)

in welcher
$R^1$ für gegebenenfalls substituiertes Cycloalkyl oder die Gruppierung

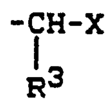 steht,

wobei
$R^3$ für Wasserstoff oder Alkyl steht und
X für Wasserstoff, Alkyl, Alkenyl, Alkinyl, Cyano, Hydroxycarbonyl, Alkoxycarbonyl, Aminocarbonyl, Alkylaminocarbonyl, Dialkylaminocarbonyl, für gegebenenfalls substituiertes Cycloalkyl, für gegebenenfalls substituiertes Aryl, für einen gebenenfalls mit einem Benzolring annellierten Heterocyclus sowie für die Gruppierung

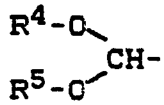

steht, wobei
$R^4$ für Alkyl steht und
$R^5$ für Alkyl steht oder
$R^4$ und $R^5$ gemeinsam mit dem Methylendioxy-Rest, an den sie gebunden sind, für einen 5-oder 6-gliedrigen Heterocyclus stehen,
$R^2$ für Wasserstoff oder Alkyl steht oder mit einem C-Atom aus der gegebenenfalls substituierten Alkylenkette A gemeinsam mit dem N-Atom, an das sie gebunden sind, für einen Heterocyclus steht, und
A für eine gegebenenfalls substituierte Alkylenkette steht,
sowie deren physiologisch verträgliche Metallsalz-Komplexe.

2. N-(2-Cyan-2-oximinoacetyl)-aminonitrile der allgemeinen Formel (I), gemäß Anspruch 1, worin
$R^1$ für gegebenenfalls ein-bis dreifach, gleich oder verschieden durch Halogen oder Methyl substituiertes Cycloalkyl mit 3 bis 6 Kohlenstoffatomen oder für die Gruppierung

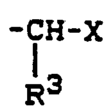 steht,

wobei
$R^3$ für Wasserstoff oder Methyl steht und

X für Wasserstoff, für geradkettiges oder verzweigtes Alkyl mit 1 bis 8 Kohlenstoffatomen, für Vinyl, Ethinyl, Cyano, Hydroxycarbonyl, für Alkoxycarbonyl mit 1 bis 4 Kohlenstoffatomen im Alkoxyteil, für Aminocarbonyl, Alkylaminocarbonyl und Dialkylaminocarbonyl mit jeweils 1 bis 4 Kohlenstoffatomen in jedem Alkylteil, für gegebenenfalls ein-oder zweifach, gleich oder veschieden, durch Halogen substituiertes Cyclopropyl, für gegebenenfalls durch Halogen oder Alkyl mit 1 bis 4 Kohlenstoffatomen substituiertes Phenyl, für einen gegebenenfalls mit einem Benzolring annellierten 5-oder 6-gliedrigen Heterocyclus, welcher 1 bis 3 gleiche oder verschiedene Heteroatome aus der Gruppe Stickstoff, Sauerstoff und Schwefel enthält sowie für den Rest

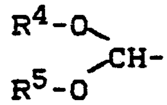 steht, wobei

$R^4$ für Methyl oder Ethyl steht und
$R^5$ für Methyl oder Ethyl steht oder
$R^4$ und $R^5$ gemeinsam mit dem Methylendioxy-Rest, an den sie gebunden sind, für einen 5-oder 6-gliedrigen Heterocyclus stehen,
$R^2$ für Wasserstoff sowie für geradkettiges oder verzweigtes Alkyl mit 1 bis 4 Kohlenstoffatomen steht oder
$R^2$ mit einem C-Atom aus der gegebenenfalls substituierten Alkylenkette A gemeinsam mit dem N-Atom, an das sie gebunden sind, für einen 5-oder 6-gliedrigen Heterocyclus steht und
A für eine gegebenenfalls bis zu dreifach, gleich oder verschieden substituierte Alkylenkette mit 1 bis 5 Kohlenstoffatomen steht wobei als Substituenten genannt seien: gegebenenfalls bis zu vierfach, gleich oder verschieden, substituiertes geradkettiges oder verzweigtes Alkyl mit 1 bis 8 Kohlenstoffatomen, wobei als Substituenten Halogen, Hydroxy, sowie Alkoxy mit 1 bis 4 Kohlenstoffatomen in Frage kommen, ferner gegebenenfalls im Arylteil einfach bis vierfach, gleich oder verschieden substituiertes Aryl oder Aralkyl mit 1 bis 4 Kohlenstoffatomen im Alkylteil und 6 bis 10 Kohlenstoffatomen in Arylteil, wobei als Substituenten Halogen, Hydroxy, Nitro sowie Alkyl und Alkoxy mit jeweils 1 bis 4 Kohlenstoffatomen genannt seien, ferner jeweils gegebenenfalls durch Alkyl mit 1 bis 4 Kohlenstoffatomen substituiertes Cycloalkyl mit 3 bis 7 Kohlenstoffatomen oder Cycloalkenyl mit 4 bis 7 Kohlenstoffatomen, sowie ein 5-oder 6-gliedriger Heterocyclus, welcher 1 bis 3 gleiche oder verschiedene Heteroatome aus der Gruppe Stickstoff, Sauerstoff und Schwefel enthält.

3. N-(2-Cyan-2-oximinoacetyl)-aminonitrile der allgemeinen Formel I gemäß Anspruch 1, worin
$R^1$ für gegebenenfalls ein-oder zweifach, gleich oder verschieden durch Chlor oder Methyl substituiertes Cyclopentyl oder Cyclohexyl oder für die Gruppierung

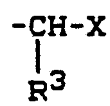 steht,

wobei
$R^3$ für Wasserstoff oder Methyl steht und
X für Wasserstoff, Methyl, Ethyl, Vinyl, Ethinyl, Cyano, Hydroxycarbonyl, Methoxycarbonyl, Ethoxycarbonyl, tert.-Butoxycarbonyl, Aminocarbonyl, Methylaminocarbonyl, Dimethylaminocarbonyl, für gegebenenfalls ein-oder zweifach durch Chlor substituiertes Cyclopropyl, für gegebenenfalls durch Chlor, Methyl, oder Ethyl substituiertes Phenyl, für Pyrazol-1-yl, 1,2,4-Triazol-1-yl, für die Reste

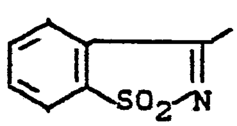 und 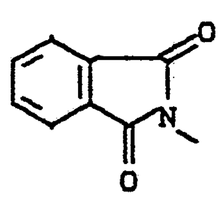 sowie

für den Rest

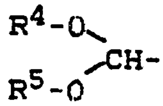

steht, wobei
$R^4$ für Methyl oder Ethyl steht und
$R^5$ für Methyl oder Ethyl steht oder
$R^4$ und $R^5$ gemeinsam mit dem Methylendioxy-Rest, an den sie gebunden sind, für einen 5-oder 6-gliedrigen Heterocyclus stehen,
$R^2$ für Wasserstoff oder Methyl steht oder
$R^2$ mit einem C-Atom der gegebenenfalls substituierten Alkylenkette A gemeinsam mit dem N-Atom, an das sie gebunden sind, für einen 5-oder 6-gliedrigen Heterocyclus steht und
A für gegebenenfalls einfach substituiertes Methylen oder Ethylen steht, wobei als Substituenten insbesondere genannt seien: gegebenenfalls einfach substituiertes geradkettiges oder verzweigtes Alkyl mit 1 bis 4 Kohlenstoffatomen, wobei als Substituenten Chlor, Brom, Hydroxy sowie Methoxy in Frage kommen, ferner jeweils gegebenenfalls durch Methyl oder Ethyl substituiertes Cyclopropyl, Cyclopentyl, Cyclohexyl, Cyclopentenyl und Cyclohexenyl, ferner 2-, 3-oder 4-Pyridyl, 2-oder 3-Thienyl sowie 2-oder 3-Furyl.

4. Verfahren zur Herstellung von N-(2-Cyan-2-oximinoacetyl)-aminonitrilen der allgemeinen Formel (I)

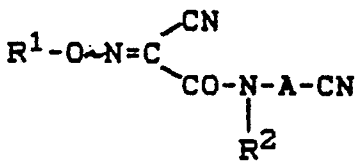 (I)

in welcher
$R^1$ für gegebenenfalls substituiertes Cycloalkyl oder die Gruppierung

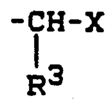 steht,

wobei
$R^3$ für Wasserstoff oder Alkyl steht und
X für Wasserstoff, Alkyl, Alkenyl, Alkinyl, Cyano, Hydroxycarbonyl, Alkoxycarbonyl, Aminocarbonyl, Alkylaminocarbonyl, Dialkylaminocarbonyl, für gegebenenfalls substituiertes Cycloalkyl, für gegebenenfalls substituiertes Aryl, für einen gegebenenfalls mit einem Benzolring annellierten Heterocyclus oder für die Gruppierung

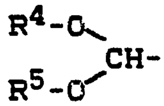

steht, wobei $R^4$ für Alkyl steht und
$R^5$ für Alkyl steht oder
$R^4$ und $R^5$ gemeinsam mit dem Methylendioxy-Rest, an den sie gebunden sind, für einen 5-oder 6-gliedrigen Heterocyclus stehen,
$R^2$ für Wasserstoff oder Alkyl steht oder mit einem C-Atom aus der gegebenenfalls substituierten Alkylenkette A gemeinsam mit dem N-Atom, an das sie gebunden sind, für einen Heterocyclus steht, und
A für eine gegebenenfalls substituierte Alkylenkette steht,
dadurch gekennzeichnet, daß man
a) carboxy-aktivierte Derivate der (E)-2-Cyan-2-oximino-essigsäuren der allgemeinen Formel (II)

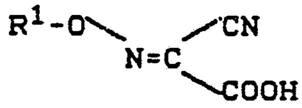 (II)

in welcher
$R^1$ die oben angegebene Bedeutung hat,
mit Aminonitrilen der allgemeinen Formel (III)

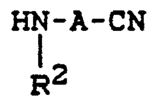 (III)

in welcher
$R^2$ und A die oben angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt,
oder
b) E-Oxime der allgemeinen Formel (IV)

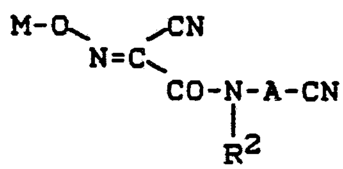 (IV)

in welcher
$R^2$ und A die oben angegebenen Bedeutungen haben und
M für Wasserstoff oder ein Alkalimetall, wie Natrium oder Kalium steht,
mit Verbindungen der allgemeinen Formel (V)
$R^1$-W (V)
in welcher
$R^1$ die oben angegebene Bedeutung hat und
W für Halogen oder einen Sulfonyl (di)oxy-Rest steht,
gegebenenfalls in Gegenwart einer Base und in Gegenwart eines Lösungsmittels umsetzt,
oder
c) (Z)-2-Cyan-2-oximino-essigsäurederivate der allgemeinen Formel (VI)

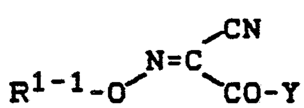 (VI)

in welcher
$R^{1-1}$ für gegebenenfalls substituiertes Cycloalkyl oder die Gruppierung

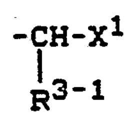

steht, wobei
$R^{3-1}$ für Wasserstoff oder Alkyl steht und
$X^1$ für Wasserstoff, Alkyl, Alkenyl, Alkinyl, Cyano, für gegebenenfalls substituiertes Cycloalkyl, für gegebenenfalls substituiertes Aryl sowie für einen gegebenenfalls mit einem Benzolring annellierten Heterocyclus steht, und
Y für Chlor oder Alkoxy steht,
mit Aminonitrilen der allgemeinen Formel (III)

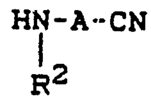

(III)

in welcher
$R^2$ und A die oben angegebenen Bedeutungen haben,
gegebenenfalls in Gegenwart eines Katalysators, gegebenenfalls in Gegenwart eines Säurebindemittels und gegebenenfalls in Gegenwart eines Verdünnungsmittels umsetzt,
oder
d) N-[(E)-2-Cyan-2-oximinoacetyl]-aminonitrile der allgemeinen Formel (I-3)

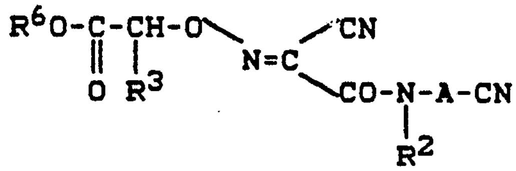

(I-3)

in welcher
$R^2$, $R^3$ und A die oben angegebenen Bedeutungen haben und
$R^6$ für Alkyl steht
mit Alkali-oder Erdalkalihydroxid in einem Verdünnungsmittel hydrolysiert,
oder
e) N-[(E)-2-Cyan-2-oximinoacetyl]-aminonitrile der allgemeinen Formel (I-3)

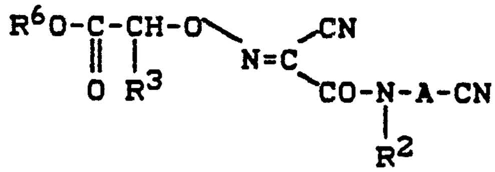

(I-3)

in welcher
$R^2$, $R^3$, $R^6$ und A die oben angegebenen Bedeutungen haben,
mit Aminen der allgemeinen Formel (VII)
$H_2N-R^7$ (VII)
in welcher $R^7$ für Wasserstoff oder Alkyl steht,
in Gegenwart eines Lösungsmittels umsetzt,
oder
f) N-[(E)-2-Cyan-2-oximinoacetyl]-aminonitrile der allgemeinen Formel (I-5)

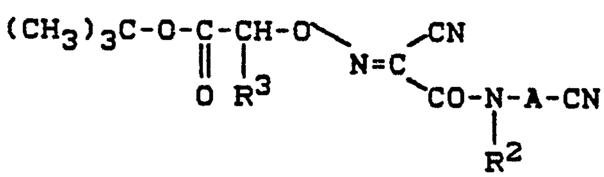

(I-5)

in welcher
$R^2$, $R^3$ und A die oben angegebenen Bedeutungen haben,
mit einer Säure, gegebenenfalls in Gegenwart eines Lösungsmittels behandelt,
oder
g) carboxy-aktivierte Derivate von N-[(E)-2-Cyan-2-oximinoacetyl]-aminonitrilen der allgemeinen Formel (I-2)

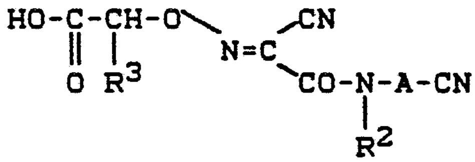

(I-2)

in welcher

$R^2$, $R^3$ und A die oben angegebenen Bedeutungen haben,

mit Aminen der allgemeinen Formel (VIII)

$HNR^8R^9$ jeweils unabhängig voneinander Wasserstoff oder Alkyl bedeuten,

gegebenenfalls in Gegenwart einer Base, in Gegenwart eines Verdünnungsmittels und gegebenenfalls in Gegenwart eines Katalysators umsetzt,

und an die so erhaltenen Verbindungen der allgemeinen Formel (I) gegebenenfalls anschließend ein Metallsalz addiert.

5. Fungizides Mittel, gekennzeichnet durch einen Gehalt an mindestens einem N-(2-Cyan-2-oximinoacetyl)-aminonitril der allgemeinen Formel I gemäß Anspruch 1.

6. Verfahren zur Bekämpfung von pilzlichen Schädlingen, dadurch gekennzeichnet, daß man N-(2-Cyan-2-oximinoethyl)-aminonitrile der allgemeinen Formel I gemäß Anspruch 1 auf pilzliche Schädlinge und/oder ihren Lebensraum einwirken läßt.

7. Verwendung von N-(2-Cyan-2-oximinoethyl)-aminonitrilen der allgemeinen Formel I gemäß Anspruch 1 zur Bekämpfung von pilzlichen Schädlingen.

8. Verfahren zur Herstellung von fungiziden Mitteln, dadurch gekennzeichnet, daß man N-(2-Cyan-2-oximinoethyl)-aminonitrile der allgemeinen Formel I gemäß Anspruch 1 mit Streckmitteln und/oder oberflächenaktiven Mitteln vermischt.

9. E-Oxime der allgemeinen Formel (IV)

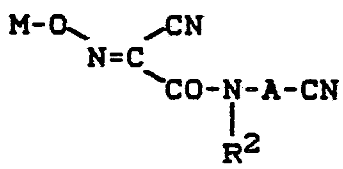

(IV)

in welcher

$R^2$ für Wasserstoff oder Alkyl steht oder mit einem C-Atom aus der gegebenenfalls substituierten Alkylenkette A gemeinsam mit dem N-Atom, an das sie gebunden sind, für einen Heterocyclus steht, und

A für eine gegebenenfalls substituierte Alkylenkette steht,

M für Wasserstoff oder ein Alkalimetall, wie Natrium oder Kalium steht.

10. Verfahren zur Herstellung von E-Oximinen der allgemeinen Formel (IV)

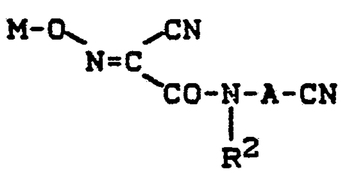

(IV)

in welcher

$R^2$ für Wasserstoff oder Alkyl steht oder mit einem C-Atom aus der gegebenenfalls substituierten Alkylenkette A gemeinsam mit dem N-Atom, an das sie Gebunden sind, für einen Heterocyclus steht, und

A für eine gegebenenfalls substituierte Alkylenkette steht,

M für Wasserstoff oder ein Alkalimetall, wie Natrium oder Kalium steht, dadurch gekennzeichnet, daß man Verbindungen der Formel (XII),

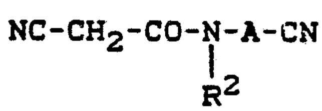

(XII)

in welcher
$R^2$ und A die oben angegebene Bedeutung haben,
mit salpetriger Säure oder Derivaten der salpetrigen Säure gegebenenfalls in Gegenwart eines Verdünnungsmittels und gegebenenfalls in Gegenwart einer Säure oder einer Base umsetzt.

Europäisches Patentamt

# EUROPÄISCHER RECHERCHENBERICHT

Nummer der Anmeldung

EP 87 11 0468

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 000 023 (BAYER)<br>* Ansprüche *<br>--- | 1-9 | C 07 C 131/00<br>A 01 N 37/50 |
| P,X | EP-A-0 201 999 (I.C.I.)<br>* Ansprüche; Tabelle I, Verbindungen 20-22,27-37,43-46,48,49,51-53,58-78,81,83-99,102-106; Seiten 24-27,30,31,40 *<br>----- | 1-10 | |
| | | | **RECHERCHIERTE SACHGEBIETE (Int. Cl.4)** |
| | | | C 07 C 131/00 |

Der vorliegende Recherchenbericht wurde für alle Patentansprüche erstellt

| Recherchenort | Abschlußdatum der Recherche | Prüfer |
|---|---|---|
| DEN HAAG | 19-11-1987 | WRIGHT M.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument